(19) Europäisches Patentamt

European Patent Office

Office européen des brevets

(11) **EP 4 674 964 A1**

(12) **EUROPEAN PATENT APPLICATION**

(43) Date of publication:
**07.01.2026 Bulletin 2026/02**

(21) Application number: **24185813.3**

(22) Date of filing: **01.07.2024**

(51) International Patent Classification (IPC):
***C12N 15/113*** (2010.01)   ***A61K 31/7088*** (2006.01)

(52) Cooperative Patent Classification (CPC):
(C-Sets available)
**C12N 15/113; A61K 31/7105; A61K 31/713;**
C12N 2310/14; C12N 2310/141         (Cont.)

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **Universiteit Antwerpen
2000 Antwerpen (BE)**

(72) Inventors:
• **Leen, Vendredy
2020 Antwerpen (BE)**
• **Vincent, Timmerman
2020 Antwerpen (BE)**

(74) Representative: **Nederlandsch Octrooibureau
P.O. Box 29720
2502 LS The Hague (NL)**

Remarks:
The complete document including Reference
Table(s) and the Sequence Listing(s) can be
downloaded from the EPO website

(54) **RNA INTERFERENCE STRATEGIES TARGETING SMALL HEAT SHOCK PROTEIN B8**

(57)    Described herein are RNA interference (RNAi) oligonucleotides for inhibiting expression of *HSPB8* in a cell, wherein the oligonucleotide comprises a guide strand sequence that is complementary to at least 15, 16, 17, 18, or 19 contiguous bases in the 3' UTR region of the human *HSPB8* transcript (SEQ ID NO: 1).

EP 4 674 964 A1

(52) Cooperative Patent Classification (CPC): (Cont.)

C-Sets
C12N 2310/14, C12N 2310/531

**Description**

**Field**

Aspects and embodiments described herein relate to the field of medicine, specifically to the field of RNA interference (RNAi) as a therapeutic tool. Aspects and embodiments described herein are useful in the treatment of diseases associated with heat shock protein B8 (HSPB8), including motor neuropathies, axonal Charcot-Marie-Tooth (CMT) neuropathies, and distal myopathies.

**Background**

Distal hereditary motor neuropathies (dHMN) or distal spinal muscular atrophies (dSMA) are a group of clinically and genetically heterogeneous diseases characterized by degeneration of motor neurons in the peripheral nervous system. Patients experience progressive motor impairment, weakness and atrophy of distal limb muscles, suggesting a length-dependent neurodegeneration affecting primarily the longest motor axons. Sensory neurons are usually not involved in dHMN. In contrast, Charcot-Marie-Tooth disease (CMT) or hereditary motor and sensory neuropathy (HMSN) patients present with both motor and sensory symptoms. However, only minor sensory involvement is recognized in the axonal form of CMT (CMT type 2 or CMT2), making it difficult to distinguish dHMN from CMT2. Intriguingly, genetic overlap is observed between CMT, dHMN and distal myopathies as these diseases can be caused by mutations in the same gene and even by the same mutation. An example of such a multiple disease-causing gene is *HSPB8,* encoding the small heat shock protein B8 (HSPB8/Hsp22) (Ghaoui et al. 2016).

HSPB8 belongs to the family of small heat shock proteins (HSPBs), a group of proteins operating as molecular chaperones that maintain cellular proteostasis (Tedesco et al. 2023). HSPB8 is ubiquitously expressed, although at different levels depending on the cell type, and an altered expression pattern has been associated with several neurodegenerative diseases such as amyotrophic lateral sclerosis, Huntington's disease and spinocerebellar ataxia. HSPB8 acts as a chaperone in association with the co-chaperone BAG3 (BCL2 associated athanogene 3), and their role in chaperone-assisted selective autophagy (CASA) together with HSC70 (heat shock cognate 70) and CHIP (C-terminus of HSC70-Interacting Protein), is well described (Ghaoui et al. 2016). Furthermore, the HSPB8-BAG3-HSC70 chaperone complex helps to ensure the integrity and dynamism of stress granules. Unrelated to the classical chaperone model, the HSPB8-BAG3 complex was reported to be involved in the phosphorylation of eIF2$\alpha$ (eukaryotic initiation factor 2 alpha), and in the remodelling of actin-based mitotic structures.

Current therapies for diseases associated with HSPB8, such as motor neuropathies, axonal CMT neuropathies, and distal myopathies, are mostly based on symptom management and include physical therapy, occupational therapy, and orthopedic devices. During past decades, many attempts have been made to develop therapeutic strategies for one or more CMT subtypes, with only a few reaching clinical trials and so far none has been approved (Stavrou et al. 2021).

**Summary**

In view of the above, there is still a need for therapeutic strategies targeting the primary genetic cause of diseases associated with HSPB8. As is described in detail below, and as is demonstrated in the experimental section herein, the current inventors have developed RNA interference (RNAi) oligonucleotides capable of inhibiting *HSPB8* expression and offering therapeutic benefits in human patient-derived cell lines as well as a mouse disease model. Accordingly, the aspects and embodiments of the present invention as described herein solve at least some of the problems and needs as discussed herein.

An aspect of the invention relates to an RNA interference (RNAi) oligonucleotide for inhibiting expression of *HSPB8* in a cell, wherein the oligonucleotide comprises a guide strand sequence that is complementary to at least 15, 16, 17, 18, or 19 contiguous bases in the 3' UTR region of the human *HSPB8* transcript (SEQ ID NO: 1). In some embodiments, the guide strand sequence is complementary to at least 15, 16, 17, 18, or 19 contiguous bases in the base sequence 5'-AGGAACCAAGCAAAGGCCAG-'3 (SEQ ID NO: 2).

In some embodiments, the oligonucleotide further comprises a passenger strand sequence that shares a region of complementarity of at least 15, 16, 17, 18, or 19 bases with the guide strand.

In some embodiments, the RNA interference (RNAi) oligonucleotide of the invention is such that it is a single stranded siRNA or a double stranded siRNA, preferably wherein the oligonucleotide comprises or consists of the base sequence of SEQ ID NO: 2 or 3. In some embodiments of an RNA interference (RNAi) oligonucleotide of the invention which is a single stranded siRNA or a double stranded siRNA, the length of the oligonucleotide is from 19 to 30 nucleotides.

In some embodiments, the RNA interference (RNAi) oligonucleotide of the invention is such that it is a short hairpin RNA (shRNA) or an artificial microRNA (amiRNA), preferably such that it comprises or consists of the sequence of SEQ ID NO: 5. In some embodiments of an RNA interference (RNAi) oligonucleotide of the invention which is a shRNA or an amiRNA, the length of the oligonucleotide is from 40 to 80, preferably from 50 to 70 nucleotides.

In some embodiments, the RNA interference (RNAi) oligonucleotide of the invention is such that it comprises one or more modified or artificial internucleoside linkages, one or more modified or artificial sugars, and/or one or more modified or artificial bases.

A further aspect of the invention relates to an expression vector encoding an oligonucleotide of the invention. In some embodiments, the expression vector is a viral vector, preferably an adeno-associated viral (AAV) vector.

A further aspect of the invention relates to a pharmaceutical composition comprising an oligonucleotide of the invention or an expression vector of the invention.

A further aspect of the invention relates to an oligonucleotide of the invention, an expression vector of the invention, or a pharmaceutical composition of the invention, for use in medicine.

A further aspect of the invention relates to an oligonucleotide of the invention, an expression vector of the invention, or a pharmaceutical composition of the invention, for use in treating or preventing a disease selected from the group consisting of a motor neuropathy, an axonal CMT neuropathy, and a distal myopathy, optionally wherein the disease is associated with a mutation in the *HSPB8* gene. In some embodiments, the motor neuropathy is distal hereditary motor neuropathy (dHMN), the axonal CMT neuropathy is Charcot-Marie-Tooth disease subtype 2L (CMT2L), and the distal myopathy is myofibrillar myopathy (MFM).

A further aspect of the invention relates to a method for inhibiting expression of *HSPB8* in a cell, said method comprising contacting the cell with an oligonucleotide of the invention or an expression vector of the invention, preferably wherein the method is an *in vitro* or an *ex vivo* method.

## Detailed description

Various features of the aspects and embodiments of this invention are further described below. It is noted that headings used throughout this specification are to assist navigation only and should not be interpreted as definitive, and that features described in different sections may be relevant for all aspects and embodiments described herein and may thus be combined as appropriate.

Introduction

Disease-causing variants in HSPB8 predominantly target the same Lysine amino acid residue at codon 141 (K141) in the highly conserved $\alpha$-crystallin domain. To gain a better understanding of the underlying pathomechanism, we previously generated and characterized a knock-in mouse model (Hspb8K141N/K141N) mimicking the clinical features of Charcot-Marie-Tooth subtype 2L (CMT2L) or dHMN patients affected with the Lys141Asn missense mutation. Interestingly, the homozygous knock-out mice (Hspb8-/-) did not develop any motor deficit or electrophysiological abnormalities (Bouhy et al. 2018). This is likely to be explained by; i) the absence of Hspb8 protein aggregates, and ii) the activation of compensatory mechanism mimicking the role of Hspb8 in the mouse. Indeed, molecular compensation is very likely to occur within the small heat shock protein family, as for instance multiple HSPBs can bind with BAG3, of which HSPB8 has the highest affinity (Rauch et al. 2017). Moreover, Hspb1 knock-out mice do not present with any overt phenotype either (Huang et al. 2007; Kammoun et al. 2016), further suggesting a (partial) redundancy among HSPB functions.

The absence of any significant neurodegenerative or neuromuscular symptoms in the Hspb8-/- mice supports the hypothesis that suppression or depletion of Hspb8 could be of therapeutic benefit. Within this rationale, we developed an RNA interference-based strategy to reduce the HSPB8/Hspb8 transcript levels in the human and mouse model, respectively. The HSPB8 knock-down could ameliorate the mitochondrial morphology and fragmentation in induced pluripotent stem cell (iPSC)-derived motor neurons from a HSPB8_K141N dHMN/CMT2L patient. On the other hand, in vivo targeting was achieved via single lumbar intrathecal injection of an adeno-associated virus AAV9 expression vector harbouring the RNA interference molecule driven by the neuron-specific synapsin promoter. The efficacy of this treatment was assessed with a multimodal approach by combining behavioural output every 3 months, and in vivo magnetic resonance imaging (MRI) to assess muscle volume and nerve structural changes at 12 months of age, and finally cross validate these results to neuromuscular pathophysiology in the experimental end point. Our data show that HSPB8/Hspb8 knock-down has therapeutic benefit for the treatment of diseases caused by HSPB8 gene mutations.

Oligonucleotides

An aspect of the invention relates to oligonucleotides capable of effecting RNA interference (RNAi)-mediated inhibition of expression of HSPB8 in a cell. The oligonucleotides comprise a guide strand sequence that is complementary to at least 15, 16, 17, 18, or 19 contiguous bases in a target region of an *HSPB8* transcript.

The oligonucleotides of this invention comprise a guide strand sequence that is complementary to at least 15, 16, 17, 18, or 19 contiguous bases in a target region of an *HSPB8* transcript, and are therefore capable of hybridising with the target region. "Hybridisation" as used herein typically refers to specific hybridisation and excludes non-specific hybridisation. Thus, in some embodiments, the oligonucleotides of this disclosure comprise a sequence that is capable of specifically hybridising with a target region of an *HSPB8* transcript. Preferably, hybridisation is assessed under physiological conditions in a cell as described herein, more preferably in a human cell as described herein. A sequence that is capable of hybridising with a target region of an *HSPB8* transcript is a sequence that has a certain level of complementarity with the target region. Perfect complementarity is not required, as long as the complementarity is sufficient to allow hybridisation, i.e. the formation of a double-stranded complex with the target region. Accordingly, in some embodiments, a guide strand sequence as described herein is at least 90%, 91%, 92%, 93%, 94%, 95%, 96%, 97%, 98%, 99% or 100% complementary to at least 15, 16, 17, 18, or 19 contiguous bases in a target region of an *HSPB8* transcript. 100% complementarity is preferred. In some embodiments, a guide strand sequence as described herein is perfectly complementary to at least 15, 16, 17, 18, or 19 contiguous bases in a target region of an *HSPB8* transcript, optionally except for the presence of up to 5, 4, 3, 2 or 1 mismatches.

An oligonucleotide is known in the art to be a polymeric molecule, such as a DNA and/or RNA molecule, consisting of repeating monomers. The monomeric units are typically nucleotides (RNA nucleotides or DNA nucleotides) or modified nucleotides ("nucleotide analogs"). The most common naturally occurring nucleotides in RNA are adenosine monophosphate (A), cytidine monophosphate (C), guanosine monophosphate (G), and uridine monophosphate (U). These consist of a pentose sugar ribose, a 5'-linked phosphate group which is linked via a phosphate ester, and a 1'-linked base. A nucleotide without a phosphate group is known as a nucleoside. The most common naturally occurring nucleotides in DNA are deoxyadenosine monophosphate (A), deoxycytidine monophosphate (C), deoxyguanosine monophosphate (G), and deoxythymidine monophosphate (T). These consist of a pentose sugar 2'-deoxyribose, a 5'-linked phosphate group which is linked via a phosphate ester, and a 1'-linked base. The abbreviations A, C, G, T and U as used herein may be used to refer to a nucleobase, a corresponding nucleoside, or a corresponding nucleotide. The term "nucleotide" as used herein includes both naturally occurring nucleotides as well as nucleotide analogs (described in more detail later herein), unless indicated otherwise. The term "oligonucleotide" as used herein encompasses salt forms of an oligonucleotide. In some embodiments, an oligonucleotide as described herein is an oligonucleotide salt, for example a sodium salt.

The sugar connects the base and the phosphate, and is therefore often referred to as the scaffold of the nucleotide. A modification in the pentose sugar is therefore often referred to as a scaffold modification. A sugar modification may therefore be called a scaffold modification. For severe modifications, the original pentose sugar might be replaced in its entirety by another moiety that similarly connects the base and the phosphate. Preferred sugars and scaffolds and modified sugars and scaffolds including artificial sugars and scaffolds are described later herein.

A base, sometimes called a nucleobase, may be selected from one of the natural DNA or RNA nucleobases (adenine, cytosine, guanine, thymine, and uracil). A base may also be a natural base analog ("modified base"), including artificial bases. Cytosine, thymine, and uracil are pyrimidine bases, and are generally linked to the scaffold through their 1-nitrogen. Adenine and guanine are purine bases, and are generally linked to the scaffold through their 9-nitrogen. Preferred bases and modified bases including artificial bases are described later herein.

A nucleotide is generally connected to neighbouring nucleotides through condensation of its 5'-phosphate moiety to the 3'-hydroxyl moiety of the neighbouring nucleotide monomer. Similarly, its 3'-hydroxyl moiety is generally connected to the 5'-phosphate of a neighbouring nucleotide monomer. This forms phosphodiester bonds. The phosphodiesters and the scaffold form an alternating copolymer. The bases are grafted to this copolymer, namely to the scaffold moieties. Because of this characteristic, the alternating copolymer formed by linked monomers of an oligonucleotide is often called the backbone of the oligonucleotide. Because the phosphodiester bonds connect neighbouring monomers together, they are often referred to as backbone linkages, internucleoside linkages or simply linkages. It is understood that when a phosphate group is modified so that it is instead an analogous moiety such as a phosphorothioate, such a moiety is still referred to as the "backbone linkage", "internucleoside linkage", or simply "linkage" of the monomer. This is referred to as a linkage modification. In general terms, the backbone of an oligonucleotide is thus comprised of alternating scaffolds and (backbone) linkages. Preferred linkages and linkage modifications including artificial linkages are described later herein.

In some embodiments, oligonucleotides described herein are non-naturally occurring oligonucleotides. For example, they may comprise at least one modified sugar, modified base, or modified linkage as described in more detail later herein. The presence of modifications, including linkage, sugar, and base modifications, may provide the oligonucleotide with improved stability and resistance properties to degradation by exonucleases. The presence of the modifications, including linkage, sugar, and base modifications, may also increase RNA interference (RNAi) efficiency. Furthermore, the presence of modifications may improve safety, bio-distribution, stability, cellular uptake, intracellular trafficking, target binding affinity, duplex stability, and immunogenicity compared to an oligonucleotide consisting of non-modified DNA and/or non-modified RNA nucleotides.

In some embodiments, oligonucleotides described herein are isolated oligonucleotides. The term "isolated" refers to the separation of a compound from other components present during its production. "isolated" is not meant to exclude artificial or synthetic mixtures with other compounds or materials, or the presence of impurities that do not substantially interfere with the fundamental activity, and that may be present, for example, due to incomplete purification, or the addition of stabilizers. In the context of oligonucleotides, the term "isolated" may refer to a molecule that is separated from sequences with which it is immediately contiguous in a naturally occurring sequence. For example, an "isolated" oligonucleotide may comprise a DNA molecule inserted into a vector, such as a plasmid or virus vector. In the context of oligonucleotides, the term "isolated" may also refer to synthetic oligonucleotides, i.e. chemically synthesized oligonucleotides. Chemical synthesis of oligonucleotides is routine in the art and provides rapid and inexpensive access to custom-made oligonucleotides of a desired sequence and a desired chemistry. The most common method is solid-phase synthesis using phosphoramidite chemistry. Reference is made to "Synthesis of Therapeutic Oligonucleotides". Eds: Satoshi Obika, Mitsuo Sekine. Springer; 1st ed. 2018, Singapore, incorporated herein by reference. Thus, in some embodiments, an oligonucleotide as described herein may be a synthetic oligonucleotide.

As explained above, oligonucleotides disclosed herein are capable of effecting RNA interference (RNAi)-mediated inhibition of expression of *HSPB8* in a cell. Along the same lines, they may be said to be capable of inducing silencing of *HSPB8* in cell, capable of repressing translation of *HSPB8* in a cell, capable of inducing *HSPB8* transcript degradation in a cell, or the like, optionally through induction of RNA interference (RNAi). Thus, the oligonucleotides disclosed herein may be denoted as "RNA interference (RNAi) oligonucleotides", "RNA silencing oligonucleotide", "gene silencing oligonucleotide", or the like. Throughout this disclosure, the terms "oligonucleotide", "RNA interference oligonucleotide", "gene silencing oligonucleotide", "RNA silencing oligonucleotide" and the like may be used interchangeably.

"RNA interference" or "RNAi" is an endogenous mechanism wherein short double-stranded RNA molecules induce posttranscriptional gene silencing by selective blockage or cleavage of a complementary mRNA sequence. The RNAi pathway is a naturally occurring process found in many eukaryotes and animal cells. It is initiated by the enzyme Dicer, which cleaves long double-stranded RNA (dsRNA) molecules into short double-stranded fragments of approximately 21 to 23 nucleotide siRNAs (or short interfering RNAs). Each siRNA is unwound into two single-stranded RNAs (ssRNAs), the passenger (sense) strand and the guide (antisense) strand. The passenger strand is then cleaved by the protein Argonaute 2 (Ago2). The passenger strand is degraded and the guide strand is incorporated into the RNA-induced silencing complex (RISC). The RISC assembly then binds and degrades the target mRNA. Specifically, this is accomplished when the guide strand pairs with a complementary sequence in a mRNA molecule and induces cleavage by Ago2, a catalytic component of the RISC.

Heat shock protein B8 (HspB8) is also known as heat shock protein beta 8, heat shock protein 22 (HSP22), or H11 kinase. In humans, heat shock protein B8 is encoded by the *HSPB8* gene (NCBI Gene ID: 26353 - transcript sequence: NCBI Reference Sequence NM_014365.3 - protein sequence: NCBI Reference Sequence NP_055180.1 or SEQ ID NO: 22). In mice, heat shock protein B8 is encoded by the Hspb8 gene (NCBI Gene ID: 80888 - transcript sequence: NCBI Reference Sequence NM_030704.3 - protein sequence: NCBI Reference Sequence NP_109629.1).

As is described in more detail in the experimental section, the inventors found that it is advantageous to target the 3' UTR of an *HSPB8* transcript. The 3' UTR region of the human HSPB8 transcript (NCBI Reference Sequence NM_014365.3) is provided as SEQ ID NO: 1. The 3' UTR region of the mouse HSPB8 transcript (NCBI Reference Sequence NM_030704.3) is provided as SEQ ID NO: 21.

Accordingly, an aspect of this disclosure relates to an RNA interference (RNAi) oligonucleotide for inhibiting expression of *HSPB8* in a cell, wherein the oligonucleotide comprises a guide strand sequence that is complementary to at least 15, 16, 17, 18, or 19 contiguous bases in the 3' UTR region of an *HSPB8* transcript. In some embodiments, the oligonucleotide comprises a guide strand sequence that is complementary to 15, 16, 17, 18, 19, 20 or 21 contiguous bases, preferably 19, 20, or 21 contiguous bases, in the 3' UTR region of an *HSPB8* transcript.

The HSPB8 gene and the associated transcript according to this disclosure may be a rodent (e.g. mouse or rat) sequence or a human sequence. Thus, an oligonucleotide as described herein may be such that the oligonucleotide comprises a guide strand sequence that is complementary to at least 15, 16, 17, 18, or 19 contiguous bases in the 3' UTR region of the human *HSPB8* transcript (SEQ ID NO: 1) and/or the mouse HSPB8 transcript (SEQ ID NO: 21). In some embodiments, the oligonucleotide comprises a guide strand sequence that is complementary to 15, 16, 17, 18, 19, 20 or 21 contiguous bases, preferably 19, 20, or 21 contiguous bases, in the 3' UTR region of the human *HSPB8* transcript (SEQ ID NO: 1) and/or the mouse *HSPB8* transcript (SEQ ID NO: 21).

It may be advantageous to be able to target mouse and human sequences with the same oligonucleotides. Accordingly, an oligonucleotide as described herein may be such that the oligonucleotide comprises a guide strand sequence that is complementary to at least 15, 16, 17, 18, or 19 contiguous bases in the 3' UTR region of the human *HSPB8* transcript (SEQ ID NO: 1) and the mouse *HSPB8* transcript (SEQ ID NO: 21). In some embodiments, the oligonucleotide comprises a guide strand sequence that is complementary to 15, 16, 17, 18, 19, 20 or 21 contiguous bases, preferably 19, 20, or 21 contiguous bases, in the 3' UTR region of the human *HSPB8* transcript (SEQ ID NO: 1) and the mouse *HSPB8* transcript (SEQ ID NO: 21).

Human sequences are generally preferred. Accordingly, a preferred aspect of this disclosure relates to an RNA interference (RNAi) oligonucleotide for inhibiting expression of *HSPB8* in a cell, wherein the oligonucleotide comprises a guide strand sequence that is complementary to at least 15, 16, 17, 18, or 19 contiguous bases in the 3' UTR region of the human *HSPB8* transcript (SEQ ID NO: 1). In some embodiments, the oligonucleotide comprises a guide strand sequence that is complementary to 15, 16, 17, 18, 19, 20 or 21 contiguous bases, preferably 19, 20, or 21 contiguous bases, in the 3' UTR region of the human HSPB8 transcript (SEQ ID NO: 1).

In preferred embodiments, a specific region in the 3' UTR of an HSPB8 transcript may be targeted. More particularly, in some embodiments, an RNAi oligonucleotide as described herein comprises a guide strand sequence that is complementary to at least 15, 16, 17, 18, or 19 contiguous bases in the base sequence 5'-AGGAACCAAGCAAAG GCCAG-'3 (SEQ ID NO: 2). In some embodiments, an RNAi oligonucleotide as described herein comprises a guide strand sequence that is complementary to the base sequence 5'-AGGAACCAAGCAAAGGCCAG-'3 (SEQ ID NO: 2). SEQ ID NO: 2 occurs in the 3' UTR of both human and mouse transcripts. Accordingly, RNAi oligonucleotides targeting this region may be capable of inhibiting HSPB8 expression in both mouse and human cells, preferably in human cells.

In some embodiments, an RNAi oligonucleotide as described herein comprises a guide strand sequence that is complementary to at least 15, 16, 17, 18, 19, or 20 contiguous bases in the base sequence 5'-AAGGAACCAAGCAA AGGCCAG-'3 (SEQ ID NO: 3). In some embodiments, an RNAi oligonucleotide as described herein comprises a guide strand sequence that is complementary to the base sequence 5'-AAGGAACCAAGCAAAGGCCAG-'3 (SEQ ID NO: 3). RNAi oligonucleotides targeting this region may also be capable of inhibiting HSPB8 expression in both mouse and human cells.

In the context of RNAi oligonucleotides as described herein, a cell may be a mammalian cell such as a rodent cell (e.g. mouse or rat cells) or a human cell, preferably a human cell. As is also described elsewhere herein, particular cell types may be preferred. In some embodiments, the cells are cells of the nervous system or of the (skeletal) muscle system, preferably the nervous system. Central nervous system cells include both glial cells and neurons but are are preferably neurons, more preferably motor neurons.

In some embodiments, a cell may be a stem cell, such as an induced pluripotent stem cell (iPSC).

Different types of RNA interference (RNAi) oligonucleotides have been described in the art and are encompassed by this disclosure. Notable examples include small interfering RNA (siRNA), microRNA (miRNA), and short hairpin RNA (shRNA), as well as derivatives and adaptations thereof. More details can be found for example in Ditzel, H. J., Tuttolomondo, M., & Kauppinen, S. (Eds.). (2021). Design and Delivery of siRNA Therapeutics. Methods in Molecular Biology, incorporated herein by reference.

In some embodiments, RNAi oligonucleotides described herein comprise a guide strand sequence but no passenger strand sequence. An example of such an embodiment is a single-stranded siRNA or ss-siRNA. Accordingly, in some embodiments, an RNAi oligonucleotide as described herein is an ss-siRNA. Single-stranded siRNAs are further described in, for example, Lima, W.F. et al. Cell 150, 883-894 (2012) and Yu, D. Cell 150, 895-908 (2012), both of which are incorporated herein by reference.

In preferred embodiments, RNAi oligonucleotides described herein comprise a passenger strand sequence that shares a region of complementarity with the guide strand. The complementary regions between the guide and passenger strands are capable of hybridizing and forming a double-stranded region. Thus, in some embodiments, an RNAi oligonucleotide as described herein comprises a double-stranded region. A double stranded region may be generated by hybridisation of two separate nucleic acids, each of which contains a region that is complementary with a region on the other molecule. Alternatively, a double stranded region may be within a single nucleic acid. In other words, the passenger strand sequence and the guide strand sequence may be present on the same nucleic acid molecule, or they may be present on separate nucleic acid molecules.

The complementarity between the guide strand sequence and the passenger strand sequence may be at least 90%, 91%, 92%, 93%, 94%, 95%, 96%, 97%, 98%, 99% or 100% . 100% complementarity is preferred. In some embodiments, complementarity between the guide strand sequence and the passenger strand sequence is perfect complementary except for the presence of up to 5, 4, 3, 2 or 1 mismatches.

In some embodiments, the region of complementarity is at least 4, 5, 6, 7, 8, 9, 10, 11, 12, 13, 14, or 15 bases. In some embodiments, the region of complementarity is at least 15, 16, 17, 18, or 19 bases. In some embodiments, the region of complementarity is between 15-21 bases, such as 15, 16, 17, 18, 19, 20 or 21 bases, preferably 19, 20, or 21 bases.

A prime example of an RNAi oligonucleotide that includes a passenger strand sequence is a double-stranded small interfering RNA (ds-siRNA), also known simply as a siRNA. In some embodiments, an RNAi oligonucleotide as described herein is a double-stranded siRNA (or briefly siRNA). In the case of double-stranded siRNAs, the passenger strand sequence is present on a separate nucleic acid strand compared to the the guide strand sequence. Double-stranded siRNAs are short nucleotide duplexes, typically containing asymmetric 2-nucleotide overhangs on the 3' ends. An siRNA enters the RNAi pathway at the stage where, naturally, a fully processed miRNA would enter.

In some embodiments, an oligonucleotide according to this disclosure is a single stranded siRNA or a double stranded siRNA.

Another example of an RNAi oligonucleotide that includes a passenger strand sequence is a short hairpin RNA (shRNA). In some embodiments, an oligonucleotide according to this disclosure is a short hairpin RNA (shRNA). In the case of shRNA, the passenger strand sequence is present on the same nucleic acid strand as the guide strand sequence. A typical shRNA has a length of 40-80 or 50-70 nucleotides and includes a stem-loop structure consisting of a 19 to 29 bp double-stranded region (the stem) bridged by a predominantly single-stranded region (the loop) and a dinucleotide 3' overhang. An shRNA enters the RNAi pathway upstream of a siRNA, similar to a natural pre-microRNA.

In some embodiments, the shRNA comprises or consists of the base sequence of SEQ ID NO: 5 or a sequence having up to 5, 4, 3, 2 or 1 mutation(s) compared to SEQ ID NO: 5. Mutations include additions, insertions, deletions and substitutions, preferably substitutions.

siRNAs and shRNAs have been further developed to more closely mimic the natural pathways and molecules. Such oligonucleotides typically contain an siRNA inserted in a scaffold based on a natural primary miRNA (pri-miRNA). Such engineered oligonucleotides are known in the art as artificial miRNAs (amiRNAs), or alternatively also as miRNA mimics, shRNA-miRs, pri-miRNA-like shRNAs, or miRNA-adapted shRNAs. An amiRNA enters the RNAi pathway upstream of a siRNA and a shRNA, similar to a natural pri-microRNA. In some embodiments, the miRNA scaffold is a miR30 scaffold.

siRNAs, shRNAs and amiRNAs are described in more detail in Kotowska-Zimmer et al. "Artificial miRNAs as therapeutic tools: Challenges and opportunities." Wiley Interdiscip Rev RNA. 2021 Jul;12(4):e1640, which is incorporated herein by reference.

The length of oligonucleotides as described herein is not particularly limited. Typically, oligonucleotides described herein may be longer than 10 nucleotides. For example, particularly in relation to siRNAs, an oligonucleotide as described herein may have a minimum length of 10, 11, 12, 13, 14, 15, 16, 17, 18, or 19 nucleotides. In some embodiments, particularly in relation to shRNAs or amiRNAs, oligonucleotides described herein may be longer than 40 nucleotides. For example, they may have a minimum length of 40, 41, 42, 43, 44, 45, 46, 47, 48, 49, or 50 nucleotides.

Typically, oligonucleotides described herein may be shorter than 80 nucleotides. For example, particularly in relation to shRNAs or amiRNAs, an oligonucleotide as described herein may have a maximum length of 80, 79, 78, 77, 76, 75, 74, 73, 72, 71, or 70 nucleotides. In some embodiments, particularly in relation to siRNAs, oligonucleotides described herein may be shorter than 30 nucleotides. For example, they may have a maximum length of 30, 29, 28, 27, 26, 25, 24, 23, or 22 nucleotides.

In some embodiments, an oligonucleotide as described herein has a mimum length according to the preferences described above, and a maximum length according to the preferences described above.

In some embodiments, particularly in embodiments relating to siRNAs, the length of the oligonucleotide is from 19 to 30 nucleotides. In some embodiments, particularly in embodiments relating to siRNAs, the length of the oligonucleotide is from 19 to 22 nucleotides.

In some embodiments, particularly in embodiments relating to shRNAs and amiRNAs, the length of the oligonucleotide is from 40 to 80, preferably from 50 to 70 nucleotides.

In some embodiments, an oligonucleotide of the invention comprises one or more modified or artificial internucleoside linkages, one or more modified or artificial sugars, and/or one or more modified or artificial bases.

*Internucleoside linkages and modifications*

As described above, oligonucleotides of this invention may comprise "natural" phosphodiester linkages as well as modified linkages (including artifical linkages). Combinations of distinct modified or artificial linkages within one molecule are encompassed.

In some embodiments, an oligonucleotide as described herein comprises one or more modified or artificial internucleoside linkages.

Modified or artificial linkages as disclosed herein may include modified versions of the phosphodiester present in natural DNA and RNA, such as phosphorothioate (PS), chirally pure phosphorothioate, (R)-phosphorothioate, (S)-phosphorothioate, phosphorodithioate (PS2), phosphonoacetate (PACE), phosphonoacetamide (PACA), thiophosphonoacetate (thioPACE), thiophosphonoacetamide, phosphorothioate prodrug, H-phosphonate, methyl phosphonate, methyl phosphonothioate, methyl phosphate, methyl phosphorothioate, ethyl phosphate, ethyl phosphorothioate, boranophosphate, boranophosphorothioate, methyl boranophosphate, methyl boranophosphorothioate, methyl boranophosphonate, methyl boranophosphonothioate, phosphate, phosphotriester, aminoalkylphosphotriester, and their derivatives. Among these, phosphorothioate (PS) is preferred. Modified or artificial linkages as disclosed herein may also include phosphoryl guanidines, acylphosphoramidates, sulfonylphosphoramidates, phosphoramidite, phosphoramidate, N3'→P5' phosphoramidate, phosphordiamidate, phosphorothiodiamidate, sulfamate, dimethylenesulfoxide, amide, sulfonate, siloxane, sulfide, sulfone, formacetyl, thioformacetyl, methylene formacetyl, alkenyl, methylenehydrazino, sulfonamide, amide, triazole, oxalyl, carbamate, methyleneimino (MMI), and thioacetamido nucleic acid (TANA); and their derivatives.

In some embodiments, an oligonucleotide as described herein comprises one or more phosphorothioate internucleoside linkages. Phosphorothioate linkages may provide protection against degradation by cellular nucleases. In some embodiments, an oligonucleotide as described herein may also consist of phosphorothioate internucleoside linkages. This means that all internucleoside linkages in the oligonucleotide are phosphorothioate internucleoside linkages. Alternatively, an oligonucleotide as described herein may predominantly contain phosphorothioate internucleoside linkages. For example, all except 1, 2, 3, 4, 5, 6, 7, 8, 9 or 10 internucleoside linkages may be phosphorothioate internucleoside linkages.

*Sugar or scaffold moieties and modifications*

As described above, oligonucleotides may comprise "natural" sugars or scaffolds as well as modified sugars or scaffolds (including artifical sugars or scaffolds). Combinations of distinct modified or artificial sugars or scaffolds within one molecule are encompassed.

Oligonucleotides of this disclosure may comprise RNA nucleotides (and modified RNA nucleotides or RNA nucleotide analogs), DNA nucleotides (and modified DNA nucleotides or DNA nucleotide analogs), and combinations thereof. In preferred embodiments, an oligonucleotide as described herein consists of RNA nucleotides or modifications thereof.

In some embodiments, an oligonucleotide as described herein comprises one or more modified or articial sugars.

Modified or artificial sugar (or scaffold) as described herein may include a modified version of the ribosyl moiety, such as 2'-*O*-modified RNA such as 2'-*O*-alkyl or 2'-*O*-(substituted)alkyl *e.g.* 2'-*O*-methyl, 2'-*O*-(2-cyanoethyl), 2'-*O*-(2-methoxy)ethyl (2'-MOE), 2'-*O*-(2-thiomethyl)ethyl, 2'-*O*-butyryl, 2'-*O*-propargyl, 2'-*O*-acetalester (such as e.g. Biscans et al. Bioorg. Med. Chem. 2015, 23, 5360), 2'-*O*-allyl, 2'-*O*-(2S-methoxypropyl), 2'-*O*-(*N*-(aminoethyl)carbamoyl)methyl) (2'-AECM), 2'-*O*-(2-carboxyethyl) and carbamoyl derivatives (Yamada et al. Org. Biomol. Chem. 2014, 12, 6457), 2'-*O*-(2-amino)propyl, 2'-*O*-(2-(dimethylamino)ethyl), 2'-*O*-(2-amino)ethyl, 2'-*O*-(2-(dimethylamino)ethyl); 2'-deoxy (DNA); 2'-*O*-(haloalkoxy)methyl (Arai K. et al. Bioorg. Med. Chem. 2011, 21, 6285) *e.g.* 2'-*O*-(2-chloroethoxy)methyl (MCEM), 2'-*O*-(2,2-dichloroethoxy)methyl (DCEM); 2'-*O*-alkoxycarbonyl *e.g.* 2'-*O*-[2-(methoxycarbonyl)ethyl] (MOCE), 2'-*O*-[2-(*N*-methylcarbamoyl)ethyl] (MCE), 2'-*O*-[2-(*N,N*-dimethylcarbamoyl)ethyl] (DCME), 2'-*O*-[2-(methylthio)ethyl] (2'-MTE), 2'-(ω-*O*-serinol); 2'-halo *e.g.* 2'-F, FANA (2'-F arabinosyl nucleic acid); 2',4'-difluoro-2'-deoxy; carbasugar and azasugar modifications; 3'-*O*-substituted *e.g.* 3'-*O*-methyl, 3'-*O*-butyryl, 3'-*O*-propargyl; 4'-substituted *e.g.* 4'-aminomethyl-2'-*O*-methyl or 4'-aminomethyl-2'-fluoro; 5'-subtituted *e.g.* 5'-methyl or CNA (Østergaard et al. ACS Chem. Biol. 2014, 22, 6227); and their derivatives.

Modified or artificial sugar (or scaffold) as described herein may also include include a bicyclic nucleic acid monomer (BNA) which may be a bridged nucleic acid monomer. Each occurrence of said BNA may result in a monomer that is independently chosen from the group consisting of a conformationally restricted nucleotide (CRN) monomer, a locked nucleic acid (LNA) monomer, a xylo-LNA monomer, an α-LNA monomer, an α-L-LNA monomer, a β-D-LNA monomer, a 2'-amino-LNA monomer, a 2'-(alkylamino)-LNA monomer, a 2'-(acylamino)-LNA monomer, a 2'-*N*-substituted-2'-amino-LNA monomer, a 2'-thio-LNA monomer, a (2'-O,4'-C) constrained ethyl (cEt) BNA monomer, a (2'-O,4'-C) constrained methoxyethyl (cMOE) BNA monomer, a 2',4'-BNA$^{NC}$(N-H) monomer, a 2',4'-BNA$^{NC}$(N-Me) monomer, a 2',4'-BNA$^{NC}$(N-Bn) monomer, an ethylene-bridged nucleic acid (ENA) monomer, a carba LNA (cLNA) monomer, a 3,4-dihydro-2*H*-pyran nucleic acid (DpNA) monomer, a 2'-C-bridged bicyclic nucleotide (CBBN) monomer, a heterocyclic-bridged BNA monomer (such as triazolyl or tetrazolyl-linked), an amido-bridged BNA monomer, an urea-bridged BNA monomer, a sulfonamide-bridged BNA monomer, a bicyclic carbocyclic nucleotide monomer, a TriNA monomer, an α-L-TriNA monomer, a bicyclo DNA (bcDNA) monomer, an abcDNA monomer, an F-bcDNA monomer, a tricyclo DNA (tcDNA) monomer, an F-tcDNA monomer, an oxetane nucleotide monomer, a locked PMO monomer derived from 2'-amino-LNA, a guanidine-bridged nucleic acid (GuNA) monomer, a spirocyclopropylene-bridged nucleic acid (scpBNA) monomer, and derivatives thereof.

Preferred modified or artificial sugars include 2'-O-modified sugars (such as 2'-O-methyl, 2'-O-(2-methoxy)ethyl (2'-MOE), and 2'-fluoro) and locked nucleic acids. 2'-O modifications may provide protection against degradation by cellular nucleases. Locked nucleic acids (LNAs), may increase affinity to the target RNA.

*Nucleobases and modifications*

As described above, oligonucleotides as described above may comprise "natural" nucleobases as well as modified nucleobases (including artifical bases). Combinations of distinct modified or artificial bases within one molecule are encompassed.

If such a base is a modified base or if a base analog is being used, said modified base or base analog should preferably keep the same base pair specificity as the base it replaces. "Base pairing" refers to the binding of two bases (or nucleobases) to each other by hydrogen bonds. Specifically, a nucleobase analog replacing cytosine is capable of base pairing with guanine, a nucleobase analog replacing guanine is capable of base pairing with cytosine, a nucleobase analog replacing adenine is capable of base pairing with uracil and a nucleobase analog replacing uracil is capable of base pairing with adenine.

Modified or artificial bases as used herein may include modified versions of the natural purine and pyrimidine bases (e.g. adenine, uracil, guanine, cytosine, and thymine), such as hypoxanthine (as present in e.g. inosine), orotic acid, agmatidine, lysidine, pseudouracil, 2-thiopyrimidine (e.g. 2-thiouracil, 2-thiothymine), G-clamp and its derivatives, 5-substituted pyrimidine (e.g. 5-halouracil, 5-propynyluracil, 5-propynylcytosine, 5-aminomethyluracil, 5-hydroxymethyluracil, 5-methyluracil (thymine), 5-methylcytosine, 5-aminomethylcytosine, 5-hydroxymethylcytosine, Super T), 7-deazaguanine, 7-deazaadenine, 2,6-diaminopurine, 7-aza-2,6-diaminopurine, 8-aza-7-deazaguanine, 8-aza-7-deazaadenine, 8-aza-7-deaza-2,6-diaminopurine, Super G, Super A, and N4-ethylcytosine, or derivatives thereof; N2-cyclopentylguanine (cPent-G), N2-cyclopentyl-2-aminopurine (cPent-AP), and N2-propyl-2-aminopurine (Pr-AP), or derivatives thereof; and degenerate or universal bases, like 2,6-difluorotoluene or absent bases like abasic sites (e.g. 1-deoxyribose, 1,2-dideoxyribose, 1-deoxy-2-O-methylribose; or pyrrolidine derivatives in which the ring oxygen has been replaced with nitrogen (azaribose)). Examples of derivatives of Super A, Super G and Super T can be found in US patent 6,683,173 (Epoch Biosciences), which is incorporated here by reference. In some embodiments, an oligonucleotide as described herein comprises one or more modified or artificial bases.

It is customary to combine modified and/or artifical internucleoside linkages, nucleobases and sugar or scaffold moieties, such as those described above, in the same molecule.

Expression constructs and vectors

The nucleic acid molecules (oligonucleotides) of this invention may comprise further sequence elements. Typically, further sequence elements may be sequence elements that are commonly used to aid in expressing a nucleotide sequence such as, promoters, nuclear localization signals, kozak sequences, polyA-tails, transcription terminators, and the like. When one or more of such further sequence elements are present, the nucleic acid molecules described herein may also be referred to as "nucleic acid constructs" or "gene constructs" or "expression constructs". It is understood that the different sequence elements may be "operably linked" with each other to achieve functional nucleic acid molecules.

As used herein, an "expression construct" refers to a DNA molecule comprising a region (coding region or ORF), which is transcribed into an RNA molecule in a cell, operably linked to a suitable regulatory region such as, but not limited to, a promoter and/or enhancer sequence. An expression construct will generally comprise multiple operably linked fragments, such as, a promoter, an enhancer, a 5' leader sequence, a coding region, and/or a 3' untranslated region (3'-end) e.g. comprising a polyadenylation and/or transcription termination site.

Molecular toolbox techniques for preparation of nucleic acid constructs are well-known in the art and are discussed in standard handbooks such as Ausubel et al., Current Protocols in Molecular Biology, 3rd edition (2003), John Wiley & Sons Inc and Sambrook and Green, Molecular Cloning: A Laboratory Manual, 4th Edition (2012), Cold Spring Harbor Laboratory Press; both of which are incorporated herein by reference in their entireties. Non-limiting examples of such techniques are fusion PCR, restriction digestion, Golden-gate cloning, and the like.

A promoter as described herein may be a constitutive promoter or an inducible promoter. A "constitutive" promoter is a promoter that is active in most tissues under most physiological and developmental conditions. An "inducible" (and/or "repressible") promoter is a promoter that is physiologically or developmentally regulated to be induced (and/or repressed), e.g. by the application of a chemical inducer or repressing signal.

A promoter as described herein may be a ubiquitous promoter or a tissue-specific promoter. A "ubiquitous" promoter is a promoter that is active in substantially all tissues, organs and cells of an organism. A "tissue-specific" (or "organ-specific") promoter is a promoter that is active in a specific type tissue (or organ). Tissue-specific promoters regulate expression of one or more genes (or coding sequence) primarily in one organ or tissue, but may allow detectable level ("leaky") expression in other organs or tissues as well. Leaky expression in other organs or tissues means at least one-fold, at least two-fold, at least three-fold, at least four-fold or at least five-fold lower, but still detectable expression as compared to the organ-specific or tissue-specific expression, as evaluated on the level of the mRNA or the protein by standard assays known to a person of skill in the art (e.g. qPCR, Western blot analysis, ELISA).

Preferred tissue-specific promoters according to this disclosure are CNS-specific promoters. These promoters are capable of initiating transcription in the CNS. Transcription in the CNS can be detected in relevant cells, such as neurons and/or glial cells, preferably neurons, more preferably motor neurons.

In some embodiments, the CNS-specific promoter is a neuron-specific promoter. In some embodiments, a neuron-specific promoter as described herein is a synapsin promoter, such as a synapsin I promoter, preferably a human synapsin I promoter. In some embodiments, a synapsin 1 promoter comprises, consists essentially of, or consists of a nucleotide sequence that has at least 60%, at least 61%, at least 62%, at least 63%, at least 64%, at least 65%, at least 66%, at least 67%, at least 68%, at least 69%, at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% sequence identity with SEQ ID NO: 23.

Suitable promoters also include promoters known to express short RNAs, such as RNA polymerase III promoters (e.g., the U6 and H1 promoters).

Promoters may be used in combination with an enhancer sequence, such as a CMV enhancer sequence. In some embodiments, a synapsin promoter such as the synapsin I promoter described herein is used in combination with an enhancer, such as the CMV enhancer. In some embodiments, a CMV enhancer comprises, consists essentially of, or consists of a nucleotide sequence that has at least 60%, at least 61%, at least 62%, at least 63%, at least 64%, at least 65%, at least 66%, at least 67%, at least 68%, at least 69%, at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% sequence identity with SEQ ID NO: 24.

Oligonucleotides and expression constructs as described herein can be placed in expression vectors. Thus, in another aspect there is provided an expression vector encoding any of the oligonucleotides as described herein (insofar as they do not contain modified or artificial linkages, sugars, or bases). In preferred embodiments, expression vectors disclosed herein encode shRNA or amiRNA oligonucleotides as described herein.

An "expression vector", alternatively referred to herein as "vector" or "delivery vector", refers to a molecular biology tool used to obtain expression of a coding region (such as a gene) in a host cell, for example by introducing a nucleotide sequence that is capable of effecting expression of a gene or a coding sequence in a host cell compatible with said sequence. An expression vector may be able to stabilize and remain episomal in a host cell. Alternatively, a vector may be able to integrate into a host cell's genome, for example through homologous recombination, non-homologous end-joining, or otherwise. A description of suitable cells in the context of this disclosure is provided elsewhere herein.

In preferred embodiments, the expression vector is a plasmid or a viral expression vector, preferably a viral expression vector. A description of "viral expression vector" or "viral vector" in short has been provided under the section entitled "general information".

A viral vector may be a viral vector selected from the group consisting of adenoviral vectors, adeno-associated viral vectors, retroviral vectors, and lentiviral vectors. An adenoviral vector is also known as an adenovirus derived vector, an adeno-associated viral vector is also known as an adeno-associated virus (AAV) derived vector, a retroviral vector is also known as a retrovirus derived vector and a lentiviral vector is also known as a lentivirus derived vector. A preferred viral vector is an adeno-associated viral (AAV) vector or a lentiviral vector. A more preferred viral vector is an AAV vector.

It is understood that the AAV expression vectors described herein are recombinant AAV (rAAV) vectors. These vectors comprise a recombinant viral genome encapsidated in a protein shell composed of a capsid protein derived from an AAV serotype, for example AAV1, AAV2, AAV3, AAV4, AAV5, AAV6, AAV7, AAV8, AAV9, or AAVrh10, preferably AAV9. The recombinant viral genome contains inverted terminal repeats (ITRs) derived from an AAV serotype, which may be the same or different as the AAV serotype from which the capsid proteins are derived. In some embodiments, the ITRs are derived from AAV2.

In some embodiments, a vector of this disclosure is a vector capable of infecting the cell types which are described elsewhere herein, including cells of the nervous system and of the (skeletal) muscle system. Cells of the nervous system are preferred, such as neurons and glial cells, preferably neurons, more preferably motor neurons.

Compositions

An aspect of this invention relates to a composition comprising an oligonucleotide as described herein and/or an expression vector as described herein. Preferably, the composition is a pharmaceutical composition. In some embodiments, such pharmaceutical composition comprises an oligonucleotide as described herein and/or an expression vector as described herein, and optionally further comprises one or more pharmaceutically acceptable ingredients.

In some embodiments, compositions described herein comprise a salt (e.g. a sodium salt) of the oligonucleotides of this disclosure.

As used herein, "pharmaceutically acceptable ingredients" include pharmaceutically acceptable carriers, fillers, preservatives, solubilizers, vehicles, diluents and/or excipients. Accordingly, the one or more pharmaceutically acceptable ingredients may be selected from the group consisting of pharmaceutically acceptable carriers, fillers, preservatives, solubilizers, vehicles, diluents, and excipients, preferably selected from the group consisting of excipients, vehicles, carriers, and diluents. Such pharmaceutically acceptable carriers, fillers, preservatives, solubilizers, vehicles, diluents and/or excipients may for instance be found in Remington: The Science and Practice of Pharmacy, 23rd edition. Elsevier (2020), incorporated herein by reference.

A further compound may be present in a composition of this disclosure. Said compound may help in delivery of the composition, for example delivery to cell types as described elsewhere herein. Suitable compounds in this context are: compounds capable of forming complexes, nanoparticles, micelles and/or liposomes. It is understood that these compounds are capable of delivering oligonucleotides and expression vectors as described herein, complexed or trapped in a vesicle or liposome, through a cell membrane. Many of these compounds are known in the art. Suitable compounds comprise polyethylenimine (PEI), or similar cationic polymers, including polypropyleneimine or polyethylenimine copolymers (PECs) and derivatives; synthetic amphiphiles (SAINT-18); lipofectin™, DOTAP. A person of skill in the art will know which type of formulation is the most appropriate for a composition as described herein.

In some embodiments, a composition as described herein is a liquid composition. In some embodiments, a composition as described herein is a solution. In some embodiments, the solution is an aqueous solution. In some embodiments, the solution is an isotonic aqueous solution. In some embodiments, a composition as described herein is a saline solution, preferably a buffered saline solution (for example a phosphate-buffered saline solution).

Compositions of this disclosure may be provided as dosage units, preferably single-use dosage units, for example in the form of a single-dose vial. A dosage unit, as used herein, refers to a physically discrete unit of the pharmaceutical composition appropriate for the patient undergoing treatment. The advantage of single-use dosage units is that they can be formulated without the use of preservatives. The compositions may be provided as concentrated solutions and diluted before use.

In some embodiments, a composition as described herein comprises an oligonucleotide as described herein at a concentration of from 0.5 mg/ml to 300 mg/ml or from 1 mg/ml to 200 mg/ml.

In some embodiments, a composition as described herein is formulated for delivery to the CNS. In some embodiments, a composition as described herein is formulated for delivery to muscle, preferably skeletal muscle.

In some embodiments, a composition as described herein is formulated for intravenous administration. In some embodiments, a composition as described herein is formulated for intramuscular administration. In preferred embodiments, a composition as described herein is formulated for (direct or indirect) administration to the cerebrospinal fluid (CSF). Direct administration to the cerebrospinal fluid includes, for example, intrathecal administration and intracerebroventricular administration.

Methods

An aspect of this invention relates to a method for inhibiting expression of *HSPB8* in a cell, said method comprising contacting the cell with an oligonucleotide as described herein or an expression vector as described herein.

In some embodiments, methods for inhibiting expression of *HSPB8* in a cell as disclosed herein, are performed *in vivo.* In preferred embodiments, methods for inhibiting expression of *HSPB8* in a cell as disclosed herein, are performed *in vitro* or *ex vivo.* In other words, methods for inhibiting expression of *HSPB8* in a cell as disclosed herein may be *in vitro* or *ex vivo* methods.

As described in more detail later, diseases associated with heat shock protein B8 (HSPB8), including motor neuropathies, axonal Charcot-Marie-Tooth (CMT) neuropathies, and distal myopathies, primarily affect cells of the nervous system. Indeed, while HSPB8 is found in cells throughout the body, it is particularly abundant in nerve cells. Apart form the nervous system, also the (skeletal) muscle system is affected. Methods for inhibiting expression of *HSPB8* in a cell as disclosed herein are preferably performed on cells expressing *HSPB8.* Methods for inhibiting expression of *HSPB8* in a cell as disclosed herein are therefore preferably performed on cells of the nervous system or of the (skeletal) muscle system, preferably the nervous system. Central nervous system cells include both glial cells and neurons but are are preferably neurons, more preferably motor neurons.

In some embodiments, methods for inhibiting expression of *HSPB8* in a cell as disclosed herein are performed in stem cells, such as induced pluripotent stem cells (iPSCs).

The cells are preferably mammalian cells such as rodent cells (e.g. mouse or rat cells) or human cells, more preferably human cells.

In some embodiments, inhibiting expression of *HSPB8* in a cell according to the methods described herein results in decreased mRNA and/or protein levels relative to an untreated state.

In some embodiments, the decrease in mRNA and/or protein levels relative to an unedited state may be 5%, 10%, 15%, 20%, 25%, 30%, 35%, 40%, 45%, or 50%. Without wishing to be bound by theory, it is expected that reduced HSPB8 levels will not lead to negative side effects, since molecular compensation by other small heat shock proteins or co-chaperones may occur.

In some embodiments, the methods for inhibiting expression of *HSPB8* in a cell as disclosed herein further comprise a step of introducing the oligonucleotide into the cell. Methods for introducing nucleic acids or derivatives thereof into host cells are commonly known to the skilled person using standard molecular techniques, as discussed in standard handbooks such as Current Protocols in Molecular Biology (Ausubel et al.), 3rd edition (2003), John Wiley & Sons, Inc (US) (incorporated herein by reference) and Sambrook and Green, Molecular Cloning. A Laboratory Manual, 4th Edition (2012), Cold Spring Harbor Laboratory Press (incorporated herein by reference).

In some embodiments, the methods for inhibiting expression of *HSPB8* in a cell as disclosed herein further comprise a step of measuring the decrease in expression. Means and methods for doing so are well-known to the skilled person. For example, expression may be assessed by measuring the levels of transgene expression in the transduced cell or tissue on the level of the mRNA or the protein by standard assays known to a person of skill in the art, such as qPCR, RNA sequencing, Northern blot analysis, Western blot analysis, mass spectrometry analysis of protein-derived peptides or ELISA.

Therapeutic methods and uses

An aspect of this invention relates to oligonucleotides, expression vectors, or pharmaceutical compositions as described herein, for use in medicine.

As described elsewhere herein, *HSPB8* mutations are known to cause motor neuropathies, axonal CMT neuropathies, and distal myopathies. Accordingly, in some embodiments, oligonucleotides, expression vectors, or pharmaceutical compositions as described herein are for use in treating or preventing a disease selected from the group consisting of a motor neuropathy, an axonal CMT neuropathy, and a distal myopathy.

It is also encompassed herein to treat or prevent symptoms associated with any of the diseases discussed herein. Thus, in some embodiments, oligonucleotides, expression vectors, or pharmaceutical compositions as described herein are for use in treating or preventing a disease selected from the group consisting of a motor neuropathy, an axonal CMT neuropathy, a distal myopathy, and symptoms thereof.

In a further aspect there is provided a method of treatment or prevention of a disease selected from the group consisting of a motor neuropathy, an axonal CMT neuropathy, and a distal myopathy (or a symptom thereof), comprising administering an oligonucleotide, expression vector, or composition as described herein. In some embodiments, administering an oligonucleotide, expression vector, or composition means administering the same to a subject, such as a subject in need thereof. In a preferred embodiment, a therapeutically (and/or prophylactically) effective amount of an oligonucleotide, expression vector, or composition is administered.

In a further aspect there is provided a use of an oligonucleotide, expression vector, or composition as described herein, for the manufacture of a medicament for the treatment or prevention of a disease selected from the group consisting of a motor neuropathy, an axonal CMT neuropathy, and a distal myopathy (or a symptom thereof).

In a further aspect there is provided a use of an oligonucleotide, expression vector, or composition as described herein, for the treatment or prevention of a disease selected from the group consisting of a motor neuropathy, an axonal CMT neuropathy, and a distal myopathy (or a symptom thereof).

Oligonucleotides, expression vectors, and compositions disclosed herein are capable of inhibiting *HSPB8* expression. Accordingly, a motor neuropathy, axonal CMT neuropathy, or distal myopathy described herein is optionally associated with mutations in the *HSPB8* gene. RNAi oligonucleotides disclosed herein are preferably targeting the 3' UTR of an *HSPB8* transcript, meaning that it is a mutation-independent approach applicable to various disease-causing *HSPB8* mutations.

In an aspect, therapeutic methods and uses of this invention are for treating or preventing a disease associated with mutations in the *HSPB8* gene. In some embodiments, a disease associated with *HSPB8* mutations is a neuromuscular disease associated with *HSPB8* mutations.

*HSPB8* mutations are preferably gain-of-function mutations. In some embodiments, the HSPB8 mutation is a mutation in the alpha-crystallin domain (ACD). In the human HSPB8 sequence of SEQ ID NO: 22, the alpha-crystallin domain corresponds with amino acids 80-170. In some embodiments, the HSPB8 mutation is a mutation of the lysine at the position corresponding to position 141 in human HSPB8 (SEQ ID NO: 22). In some embodiments, the HSPB8 mutation is a substitution of the lysine at the position corresponding to position 141 in human HSPB8 (SEQ ID NO: 22), preferably a substitution of lysine for arginine (K141N).

In some embodiments, a motor neuropathy, optionally a motor neuropathy associated with mutations in the *HSPB8* gene, is distal hereditary motor neuropathy (dHMN). In some embodiments, an axonal CMT neuropathy, optionally an axonal CMT neuropathy associated with mutations in the *HSPB8* gene, is Charcot-Marie-Tooth disease subtype 2L (CMT2L). In some embodiments, a distal myopathy, optionally a distal myopathy associated with mutations in the HSP88gene, is myofibrillar myopathy (MFM). Accordingly, in some embodiments, therapeutic methods and uses of this invention are for treating or preventing a disease selected from the group consisting of distal hereditary motor neuropathy (dHMN), Charcot-Marie-Tooth disease subtype 2L (CMT2L), and myofibrillar myopathy (MFM).

Within the context of oligonucleotides for use, expression vectors for use, compositions for use, methods and uses according to the invention, an effective amount or therapeutically (and/or prophylactically) effective amount may be administered.

As used herein, an "effective amount" is an amount sufficient to exert beneficial or desired results. Accordingly, a "therapeutically effective amount" (prophylactically effective amount) is an amount that, when administered to a subject in need thereof, is sufficient to exert some therapeutic (prophylactic) effect as described herein, such as, but not limited to, decreasing HSPB8 protein levels, optionally compared to an untreated subject. An amount that is "therapeutically effective" (and/or prophylactically effective) will vary from subject to subject, depending on the age, the disease progression and overall general condition of the individual. An appropriate "therapeutically effective" (and/or prophylactically effective) amount in any individual case may be determined by the skilled person using routine experimentation, such as the methods described later herein.

Within the context of oligonucleotides for use, expression vectors for use, compositions for use, methods and uses according to the invention, the oligonucleotides, expression vectors and compositions may be administered to a subject, such as a subject in need thereof. In some embodiments, the subject (in need) can be a healthy, asymptomatic or partially symptomatic subject (for example before (full) onset of the disease). In some embodiments, the subject (in need) may suffer from any of the diseases discussed herein, or a symptom thereof, or be at risk for developing any of the diseases discussed herein, or a symptom thereof. In some embodiments, the subject (in need) may be a subject inflicted with any of the diseases discussed herein, or a symptom thereof.

The subject treated may be a vertebrate, preferably a mammal, such as a rodent (preferably mice, rats), or a human. In preferred embodiments, the subject treated is a human.

Within the context of oligonucleotides for use, expression vectors for use, compositions for use, methods and uses according to the invention, the oligonucleotides, expression vectors and compositions as described herein preferably exhibit at least one, at least two, at least three, or all of the following effects:

- decrease in *HSPB8* expression;
- alleviating a symptom of a disease described herein; and
- improving a parameter associated with a disease described herein.

In some embodiments, symptoms of the diseases described herein include distal muscle weakness and atrophy, length-dependent neurodegeneration, and degeneration of motor neurons.

Alleviating a symptom may mean that a symptom is improved or decreased or that the progression of a typical symptom has been slowed down in an individual, in a cell, tissue or organ of said individual as assessed by a physician. A decrease or improvement of a typical symptom may mean a slowdown in progression of symptom development or a complete disappearance of symptoms. Symptoms, and thus also a decrease in symptoms, can be assessed using a variety of methods known to the skilled person.

In this context, "decrease" (respectively "improvement") means at least a detectable decrease (respectively a detectable improvement) using an assay known to a person of skill in the art. The decrease may be a decrease of at least 5%, at least 10%, at least 20%, at least 30%, at least 40%, at least 50%, at least 60%, at least 70%, at least 80%, at least 90% or at least 100%. The decrease may be seen after at least one week, one month, six months, one year or more of treatment using an oligonucleotide, expression vector or composition of the invention.

In some embodiments, parameters associated with the diseases described herein are selected from:

- decreased HSPB8 protein levels;
- decreased HSPB8 transcript levels;
- decreased BAG3 protein and/or transcript levels;
- altered expression of Heat shock protein A (HSPA), chaperone DNAJ, and STUB1; and
- malfunctioning of the chaperone-assisted selective autophagy complex.

Improving a parameter may mean that the value of a typical parameter is improved in an individual or in a cell, tissue, or organ of said individual, as assessed by a physician. In this context, improvement of a parameter may be interpreted as to mean that said parameter assumes a value closer to the value displayed by a healthy individual. The improvement of a parameter may be seen after at least one week, one month, six months, one year or more of treatment using a gene construct and/or an expression vector and/or a composition of the invention.

An oligonucleotide and/or an expression vector and/or a composition as described herein may be suitable for administration to a cell, tissue and/or an organ *in vivo* of individuals affected by or at risk of developing diseases described herein. Said gene construct and/or expression vector and/or composition may be directly or indirectly administered to a cell, tissue and/or an organ *in vivo* of an individual affected by or at risk of developing diseases described herein.

Within the context of oligonucleotides for use, expression vectors for use, compositions for use, methods and uses according to the invention, an oligonucleotide and/or an expression vector and/or a composition may be administered by different administration modes. Generally speaking, an administration mode may be intravenous, intramuscular, intraperitoneal, intranasal, subcutaneous, intraarticular, intra-adipose tissue, oral, intrahepatic, intrasplanchnic, intraductal, intra-ear, intracranial, intraparenchymal, intrathecal, intracerebroventricular, intracerebral, hippocampal, striatal administration, ophthalmic administration, administration via the cerebrospinal fluid (CSF) and/or administration via the cisterna magna.

In some embodiments, an administration mode is suitable for (direct or indirect) delivery of an oligonucleotide or expression vector to the central nervous system or skeletal muscle. In some embodiments, the administration mode is intravenous. In some embodiments, an administration mode is for (direct or indirect) delivery to the cerebrospinal fluid (CSF), preferably intrathecal administration or intracerebroventricular administration. In some embodiments, an administration mode is for (direct or indirect) delivery to skeletal muscle, preferably intramuscular administration.

An oligonucleotide, expression vector or composition of the invention may be directly or indirectly administered using suitable means known in the art. Improvements in means for providing an individual or a cell, tissue, or organ of said individual with a oligonucleotide and/or an expression vector and/or a composition of the invention are anticipated, considering the progress that has already thus far been achieved. Such future improvements may of course be incorporated to achieve the effects of the invention.

"Treating" or "treatment" as used herein may include delaying, ameliorating or curing. Accordingly, throughout this disclosure, "treating" and the like may be replaced with "treating, delaying, ameliorating or curing" and the like. In particular embodiments, "treating" or "treatment" as used herein may be delaying the onset and/or progression of diseases discussed herein, or a symptom thereof.

Within the context of compositions for use, methods and uses provided herein, "treating" as used herein may be understood to treat a disease, condition or symptom as compared to said disease, condition, or symptom prior to administering the composition. In some embodiments, "treating" as used herein may be understood to treat a disease, condition or symptom as compared to said disease, condition or symptom in a subject inflicted with the same disease, condition or symptom receiving a placebo. Similarly, the occurrence of any of the effects described elsewhere herein may be compared in the same way.

**General information**

Unless stated otherwise, all technical and scientific terms used herein have the same meaning as customarily and ordinarily understood by a person of ordinary skill in the art to which this disclosure belongs, and read in view of this disclosure.

As used herein, the term "promoter" or "regulatory sequence" refers to a nucleic acid fragment that functions to control the transcription of one or more coding sequences, and is located upstream with respect to the direction of transcription of the transcription initiation site of the coding sequence, and is structurally identified by the presence of a binding site for DNA-dependent RNA polymerase, transcription initiation sites and any other DNA sequences, including, but not limited to transcription factor binding sites, repressor and activator protein binding sites, and any other sequences of nucleotides known to one of skill in the art to act directly or indirectly to regulate the amount of transcription from the promoter. A "constitutive" promoter is a promoter that is active in most tissues under most physiological and developmental conditions. An "inducible" and/or "repressible" promoter is a promoter that is physiologically or developmentally regulated to be induced and/or repressed, e.g. by the application of a chemical inducer or repressing signal.

As used herein, the term "operably linked" refers to a linkage of polynucleotide elements in a functional relationship. A nucleic acid is "operably linked" when it is placed into a functional relationship with another nucleic acid sequence. For instance, a transcription regulatory sequence sucha s a promoter is operably linked to a coding sequence if it affects the transcription of the coding sequence. Operably linked means that the DNA sequences being linked are typically contiguous and, where necessary to join two protein encoding regions, contiguous and in reading frame.

The terms "protein" or "polypeptide" are used interchangeably and refer to molecules consisting of a chain of amino acids, without reference to a specific mode of action, size, 3-dimensional structure or origin.

*Sequence identity*

It is to be understood that each nucleic acid molecule or protein fragment or polypeptide or peptide or derived peptide or construct as identified herein by a given sequence identity number (SEQ ID NO) is not limited to this specific sequence as disclosed.

Throughout this application, each time one refers to a specific nucleotide sequence SEQ ID NO (take SEQ ID NO: X as example) encoding a given protein fragment or polypeptide or peptide or derived peptide, one may replace it by:

    i. a nucleotide sequence that has at least 50%, 55%, 60%, 65%, 70%, 75%, 80%, 85%, 90%, 95%, 97% or 99% sequence identity with SEQ ID NO: X;
    ii. a nucleotide sequence the sequence of which differs from the sequence of a nucleic acid molecule of (i) due to the degeneracy of the genetic code; or
    iii. a nucleotide sequence that encodes an amino acid sequence that has at least 50%, 55%, 60%, 65%, 70%, 75%, 80%, 85%, 90%, 95%, 97% or 99% amino acid identity or similarity with an amino acid sequence encoded by a nucleotide sequence SEQ ID NO: X.

A preferred level of sequence identity or similarity is 70%. Another preferred level of sequence identity or similarity is 75%. Another preferred level of sequence identity or similarity is 80%. Another preferred level of sequence identity or similarity is 85%. Another preferred level of sequence identity or similarity is 90%. Another preferred level of sequence identity or similarity is 95%. Another preferred level of sequence identity or similarity is 97%. Another preferred level of sequence identity or similarity is 99%.

Throughout this application, each time one refers to a specific amino acid sequence SEQ ID NO (take SEQ ID NO: Y as example), one may replace it by a sequence that has at least 50%, 55%, 60%, 65%, 70%, 75%, 80%, 85%, 90%, 95%, 97% or 99% sequence identity or similarity with amino acid sequence SEQ ID NO: Y. A preferred level of sequence identity is 70%. Another preferred level of sequence identity or similarity is 75%. Another preferred level of sequence identity or similarity is 80%. Another preferred level of sequence identity or similarity is 85%. Another preferred level of sequence identity or similarity is 90%. Another preferred level of sequence identity or similarity is 95%. Another preferred level of sequence identity or similarity is 97%. Another preferred level of sequence identity or similarity is 99%.

Each nucleotide sequence or amino acid sequence described herein by virtue of its identity or similarity percentage with a given nucleotide sequence or amino acid sequence respectively has in a further preferred embodiment an identity or a similarity of at least 60%, at least 61%, at least 62%, at least 63%, at least 64%, at least 65%, at least 66%, at least 67%, at least 68%, at least 69%, at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% with the given nucleotide or amino acid sequence, respectively.

Each non-coding nucleotide sequence (e.g. of a promoter or of another regulatory region) could be replaced by a nucleotide sequence comprising a nucleotide sequence that has at least 60% sequence identity or similarity with a specific nucleotide sequence SEQ ID NO (take SEQ ID NO: A as example). A preferred nucleotide sequence has at least 60%, at least 61%, at least 62%, at least 63%, at least 64%, at least 65%, at least 66%, at least 67%, at least 68%, at least 69%, at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% identity with SEQ ID NO: A. In a preferred embodiment, such non-coding nucleotide sequence such as a promoter exhibits or exerts at least an activity of such a non-coding nucleotide sequence such as an activity of a promoter as known to a person of skill in the art.

The terms "homology", "sequence identity" and the like are used interchangeably herein. Sequence identity is described herein as a relationship between two or more amino acid (polypeptide or protein) sequences or two or more nucleic acid (polynucleotide) sequences, as determined by comparing the sequences. In a preferred embodiment, sequence identity is calculated based on the full length of two given sequences or on a part thereof, more preferably based on the full length of two given sequences. Part thereof preferably means at least 50%, 60%, 70%, 80%, 90%, or 100% of both sequences. In the art, "identity" also refers to the degree of sequence relatedness between amino acid or nucleic acid sequences, as the case may be, as determined by the match between strings of such sequences. "Similarity" between two amino acid sequences is determined by comparing the amino acid sequence and its conserved amino acid substitutes of one polypeptide to the sequence of a second polypeptide. "Identity" and "similarity" can be readily calculated by known methods, including but not limited to those described in Bioinformatics and the Cell: Modern Computational Approaches in Genomics, Proteomics and transcriptomics, Xia X., Springer International Publishing, New York, 2018; and Bioinformatics: Sequence and Genome Analysis, Mount D., Cold Spring Harbor Laboratory Press, New York, 2004, each incorporated herein by reference.

"Sequence identity" and "sequence similarity" can be determined by alignment of two peptide or two nucleotide sequences using global or local alignment algorithms, depending on the length of the two sequences. Sequences of similar lengths are preferably aligned using a global alignment algorithm (e.g. Needleman-Wunsch) which aligns the sequences optimally over the entire length, while sequences of substantially different lengths are preferably aligned using a local alignment algorithm (e.g. Smith-Waterman). Sequences may then be referred to as "substantially identical" or "essentially similar" when they (when optimally aligned by for example the program EMBOSS needle or EMBOSS water using default parameters) share at least a certain minimal percentage of sequence identity (as described below).

A global alignment is suitably used to determine sequence identity when the two sequences have similar lengths. When sequences have a substantially different overall length, local alignments, such as those using the Smith-Waterman algorithm, are preferred. EMBOSS needle uses the Needleman-Wunsch global alignment algorithm to align two sequences over their entire length (full length), maximizing the number of matches and minimizing the number of gaps. EMBOSS water uses the Smith-Waterman local alignment algorithm. Generally, the EMBOSS needle and EMBOSS water default parameters are used, with a gap open penalty = 10 (nucleotide sequences) / 10 (proteins) and gap extension penalty = 0.5 (nucleotide sequences) / 0.5 (proteins). For nucleotide sequences the default scoring matrix used is DNAfull and for proteins the default scoring matrix is Blosum62 (Henikoff & Henikoff, 1992, PNAS 89, 915-919, incorporated herein by reference).

Alternatively percentage similarity or identity may be determined by searching against public databases, using algorithms such as FASTA, BLAST, etc. Thus, the nucleic acid and protein sequences of some embodiments of the present disclosure can further be used as a "query sequence" to perform a search against public databases to, for example, identify other family members or related sequences. Such searches can be performed using the BLASTn and BLASTx programs (version 2.0) of Altschul, et al. (1990) J. Mol. Biol. 215:403-10, incorporated herein by reference. BLAST nucleotide searches can be performed with the NBLAST program, score = 100, wordlength = 12 to obtain nucleotide sequences homologous to nucleic acid molecules of the disclosure. BLAST protein searches can be performed with the BLASTx program, score = 50, wordlength = 3 to obtain amino acid sequences homologous to protein molecules of the disclosure. To obtain gapped alignments for comparison purposes, Gapped BLAST can be utilized as described in Altschul et al., (1997) Nucleic Acids Res. 25(17): 3389-3402, incorporated herein by reference. When utilizing BLAST and Gapped BLAST programs, the default parameters of the respective programs (e.g., BLASTx and BLASTn) can be used. See the homepage of the National Center for Biotechnology Information accessible on the world wide web at www.ncbi.nlm.nih.gov/.

Optionally, in determining the degree of amino acid similarity, the skilled person may also take into account so-called conservative amino acid substitutions. As used herein, "conservative" amino acid substitutions refer to the interchangeability of residues having similar side chains. Examples of classes of amino acid residues for conservative substitutions are given in the Tables below.

| Acidic Residues | Asp (D) and Glu (E) |
|---|---|
| Basic Residues | Lys (K), Arg (R), and His (H) |
| Hydrophilic Uncharged Residues | Ser (S), Thr (T), Asn (N), and Gln (Q) |
| Aliphatic Uncharged Residues | Gly (G), Ala (A), Val (V), Leu (L), and Ile (I) |
| Non-polar Uncharged Residues | Cys (C), Met (M), and Pro (P) |
| Aromatic Residues | Phe (F), Tyr (Y), and Trp (W) |

Alternative conservative amino acid residue substitution classes :

| 1 | A | S | T |
|---|---|---|---|
| 2 | D | E | |
| 3 | N | Q | |
| 4 | R | K | |
| 5 | I | L | M |
| 6 | F | Y | W |

Alternative physical and functional classifications of amino acid residues:

| Alcohol group-containing residues | S and T |
|---|---|
| Aliphatic residues | I, L, V, and M |
| Cycloalkenyl-associated residues | F, H, W, and Y |
| Hydrophobic residues | A, C, F, G, H, I, L, M, R, T, V, W, and Y |
| Negatively charged residues | D and E |
| Polar residues | C, D, E, H, K, N, Q, R, S, and T |
| Positively charged residues | H, K, and R |
| Small residues | A, C, D, G, N, P, S, T, and V |
| Very small residues | A, G, and S |
| Residues involved in turn formation | A, C, D, E, G, H, K, N, Q, R, S, P and T |
| Flexible residues | Q, T, K, S, G, P, D, E, and R |

For example, a group of amino acids having aliphatic side chains is glycine, alanine, valine, leucine, and isoleucine; a group of amino acids having aliphatic-hydroxyl side chains is serine and threonine; a group of amino acids having amide-containing side chains is asparagine and glutamine; a group of amino acids having aromatic side chains is phenylalanine, tyrosine, and tryptophan; a group of amino acids having basic side chains is lysine, arginine, and histidine; and a group of amino acids having sulphur-containing side chains is cysteine and methionine. Preferred conservative amino acids substitution groups are: valine-leucine-isoleucine, phenylalanine-tyrosine, lysine-arginine, alanine-valine, and asparagine-glutamine. Substitutional variants of the amino acid sequence disclosed herein are those in which at least one residue in the disclosed sequences has been removed and a different residue inserted in its place. Preferably, the amino acid change is conservative. Preferred conservative substitutions for each of the naturally occurring amino acids are as follows: Ala to Ser; Arg to Lys; Asn to Gin or His; Asp to Glu; Cys to Ser or Ala; Gln to Asn; Glu to Asp; Gly to Pro; His to Asn or Gin; Iie to Leu or Val; Leu to Iie or Val; Lys to Arg; Gln or Glu; Met to Leu or Iie; Phe to Met, Leu or Tyr; Ser to Thr; Thr to Ser; Trp to Tyr; Tyr to Trp or Phe; and, Val to Iie or Leu. Particularly suitable amino acid substitutions in this disclosure include the substitutioLys for Arg and Arg for Lys.

*Operably linked*

As used herein, the term "operably linked" refers to a linkage of polynucleotide elements in a functional relationship. A nucleic acid is "operably linked" when it is placed into a functional relationship with another nucleic acid sequence. For instance, a transcription regulatory sequence is operably linked to a coding sequence if it affects the transcription of the coding sequence. Operably linked means that the DNA sequences being linked are typically contiguous and, where necessary to join two protein encoding regions, contiguous and in reading frame. Linking can be accomplished by ligation at convenient restriction sites or at adapters or linkers inserted in lieu thereof, or by gene synthesis.

*Proteins and amino acids*

The terms "protein" or "peptide" or "polypeptide" or "amino acid sequence" are used interchangeably and refer to molecules consisting of a chain of amino acids, without reference to a specific mode of action, size, 3-dimensional structure or origin. In amino acid sequences as described herein, amino acids or "residues" are denoted by three-letter symbols. These three-letter symbols as well as the corresponding one-letter symbols are well known to a person of skill in the art and have the following meaning: A (Ala) is alanine, C (Cys) is cysteine, D (Asp) is aspartic acid, E (Glu) is glutamic acid, F (Phe) is phenylalanine, G (Gly) is glycine, H (His) is histidine, I (Iie) is isoleucine, K (Lys) is lysine, L (Leu) is leucine, M (Met) is methionine, N (Asn) is asparagine, P (Pro) is proline, Q (Gln) is glutamine, R (Arg) is arginine, S (Ser) is serine, T (Thr) is threonine, V (Val) is valine, W (Trp) is tryptophan, Y (Tyr) is tyrosine. A residue may be any proteinogenic amino acid, but also any non-proteinogenic amino acid such as D-amino acids and modified amino acids formed by post-translational modifications, and also any non-natural amino acid.

*Expression*

Expression may be assessed by any method known to a person of skill in the art. For example, expression may be assessed by measuring the levels of transgene expression in the transduced tissue on the level of the mRNA or the protein by standard assays known to a person of skill in the art, such as qPCR, RNA sequencing, Northern blot analysis, Western blot analysis, mass spectrometry analysis of protein-derived peptides or ELISA.

Expression may be assessed at any time after administration of the oligonucleotide, gene construct, expression vector or composition as described herein. In some embodiments herein, expression may be assessed after 1 week, 2 weeks, 3 weeks, 4, weeks, 5 weeks, 6 weeks, 7 weeks, 8 weeks, 9, weeks, 10 weeks, 11 weeks, 12 weeks, 14 weeks, 16 weeks, 18 weeks, 20 weeks, 22 weeks, 24 weeks, 28 weeks, 32 weeks, 36 weeks, 40 weeks, or more.

In this document and in its claims, the verb "to comprise" and its conjugations is used in its non-limiting sense to mean that items following the word are included or contained, but items not specifically mentioned are not excluded. Thus, the terms 'comprising', 'comprises', 'comprised of' and the like as used herein are synonymous with 'including', 'includes' or 'containing', 'contains', and are inclusive or open-ended and do not exclude additional, non-recited members, elements or method steps.

In addition, the verb "to consist" may be replaced by "to consist essentially of" meaning that a composition as described herein may comprise additional component(s) than the ones specifically identified, said additional component(s) not altering the unique characteristic of this disclosure. In addition, the verb "to consist" may be replaced by "to consist essentially of" meaning that a method as described herein may comprise additional step(s) than the ones specifically identified, said additional step(s) not altering the unique characteristic of this disclosure.

Throughout this disclosure, the term "comprising" may be replaced with the term "consisting essentially of" or "consisting of".

As used herein, the singular forms 'a', 'an', and 'the' include both singular and plural referents unless the context clearly dictates otherwise; for example, "an antibody," is understood to represent one or more antibodies. As such, the terms "a" (or "an"), "one or more," and "at least one" can be used interchangeably herein.

The recitation of numerical ranges by endpoints includes all numbers and fractions subsumed within the respective ranges, as well as the recited endpoints.

As used herein, with "at least" a particular value means that particular value or more. For example, "at least 2" is understood to be the same as "2 or more" i.e., 2, 3, 4, 5, 6, 7, 8, 9, 10, 11, 12, 13, 14, 15, ..., etc.

Furthermore, the terms first, second, third and the like in the description and in the claims, are used for distinguishing between similar elements and not necessarily for describing a sequential or chronological order. It is to be understood that the terms so used are interchangeable under appropriate circumstances and that the embodiments described herein are capable of operation in other sequences than described or illustrated herein.

The word "about" or "approximately" when used in association with a numerical value (e.g. about 10) preferably means that the value may be the given value (of 10) more or less 5%, preferably more or less 1% of the value.

As used herein, the term "and/or" indicates that one or more of the stated cases may occur, alone or in combination with at least one of the stated cases, up to with all of the stated cases.

Various embodiments are described herein. Each embodiment as identified herein may be combined together unless otherwise indicated. Titles, subtitles, headings and the likes are used herein solely for ease of reading and are not intended to limit or restrict the disclosure in any way.

All documents including patent applications, patents, and literature references cited herein are incorporated herein by reference in their entireties, except for any definitions, subject matter disclaimers or disavowals, and except to the extent that the incorporated material is inconsistent with the express disclosure herein, in which case the language in this disclosure controls.

One skilled in the art will recognize many methods and materials similar or equivalent to those described herein, which could be used in the practice of the present invention. Indeed, the present invention is in no way limited to the specific methods and materials described.

The present invention is further described by the following examples which should not be construed as limiting the scope of the invention. The generalization of certain aspects and features disclosed in the below examples to the foregoing description is part of this disclosure.

**Description of the figures**

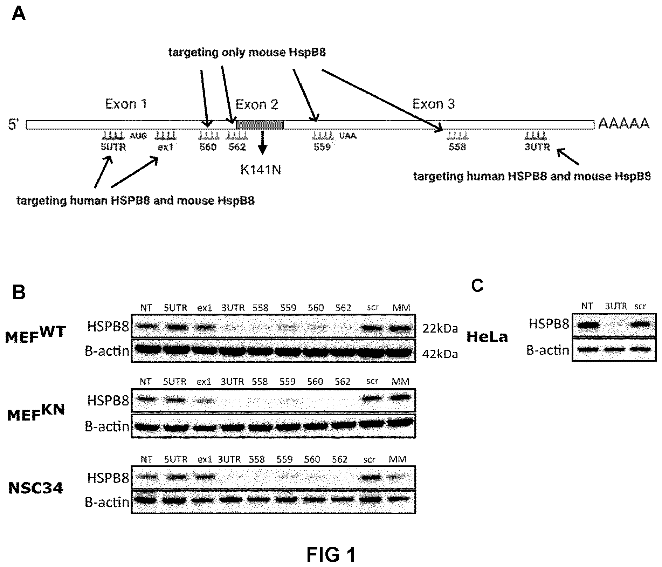

**Figure 1. Targeting shRNAs knocking-down HSPB8/Hspb8 in different cell models.** A) Schematic representation of all shRNA sequences tested, distributed over the human HSPB8 and mouse Hspb8 mRNA, and relative to the location of the K141N mutation in exon 2. B) Western blot analysis of mouse Hspb8 protein abundance in different murine cell lines stably expressing the different shRNA constructs. A scrambled and a mismatch shRNA were used as controls. NT = non-transduced, SCR = scrambled shRNA, MM = mismatch shRNA. Cell lines: MEF = mouse embryonal fibroblasts, NSC34 = mouse motor neuron cell line produced by the fusion of motor neurons from the spinal cords of mouse embryos with mouse neuroblastoma cells. C) Western blot analysis of human HSPB8 protein abundance in HeLa cells stably expressing the scrambled or 3UTR shRNA constructs. NT = non-transduced, SCR = scrambled shRNA.

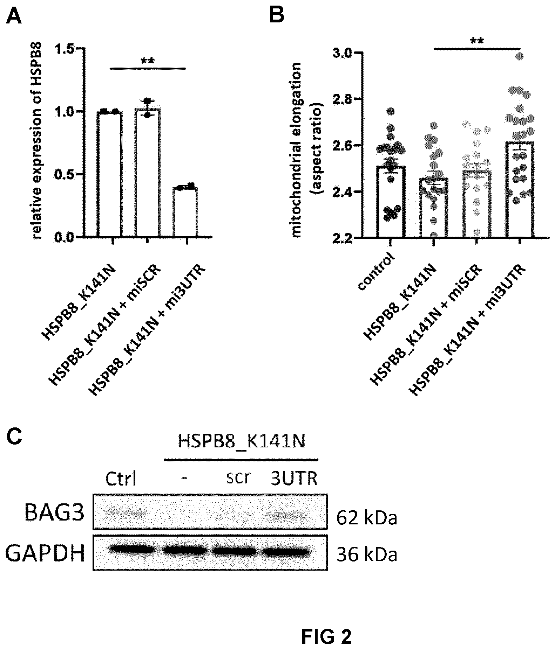

**Figure 2. Improved mitochondrial morphology upon reduction of HSPB8 expression in patient iPSC-derived motor neurons. A)** RT-qPCR showing the relative expression of HSPB8 at D37-42 in iPSC-derived motor neurons left untreated or transduced with a shRNA lentiviral construct. Data points represent data from two independent differentiations (as marked by the different symbols; mean $\pm$ SEM). Statistical significance was performed using one-way ANOVA with Dunn's multiple comparisons test (** P<0.01). **B)** Quantification of mitochondrial elongation (aspect ratio). Data-points represent the average values for the mitochondria present per image (n = 8-10; data pooled from two independent differentiations; meant SEM). Statistical significance was calculated using one-way ANOVA with Dunnetts's multiple comparison test (**P < 0.01). C) BAG3 levels at D37-42 in iPSC-derived motor neurons determined using immunoblotting. Ctrl = control, scr = scrambled.

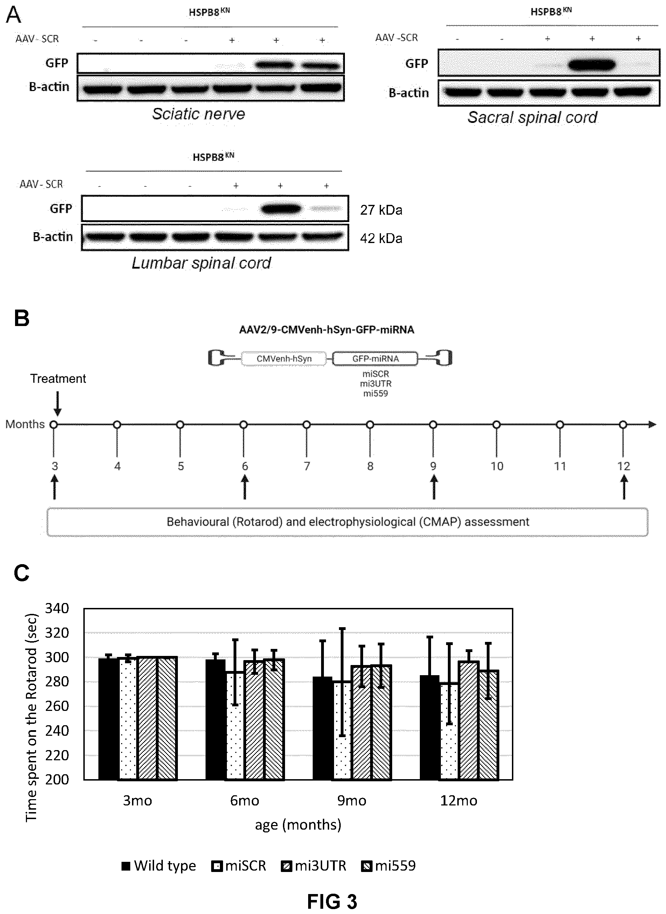

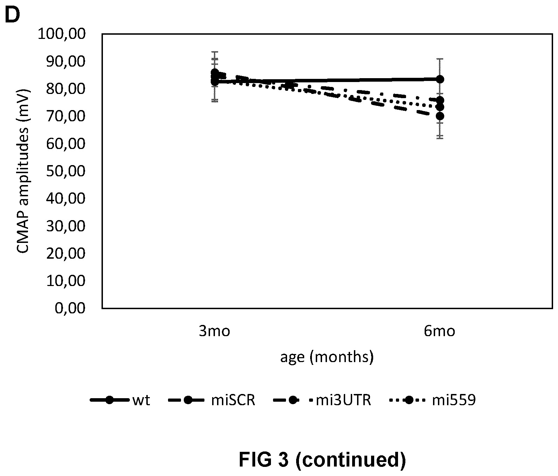

**Figure 3. Hspb8 knock-down *in vivo* holds promise for amelioration of motor performance. A)** Western blot analysis to confirm GFP expression versus β-actin staining as protein loading control, indicative of successful intrathecal injection and long-term expression of the GFP-miRNA constructs in different Hspb8$^{K141N/K141N}$ knock-in mice (pilot study). These animals were injected at 3 months of age and sacrificed 4 weeks after injection. Protein samples were taken from nerve and spinal cord after 4 weeks of treatment. - or + indicates respectively without or with AAV2/9.miHSPB8 delivery. **B)** Schematic representation of the experimental set-up for the mice study. **C)** Motor performance tested every three months by a rotarod test. Data points represent the average time spend (sec) on an accelerated rotarod per treatment group. Significance was tested using 2-way ANOVA with Dunnetts's multiple comparison test. **D)** Nerve conduction tested just before treatment and after 6 months of AAV2/9.miHSPB8 treatment by measuring compound muscle action potential amplitudes (CMAP). Data points represent the average amplitude (mV) per treatment group. Significance was tested using 2-way ANOVA with Dunnetts's multiple comparison test.

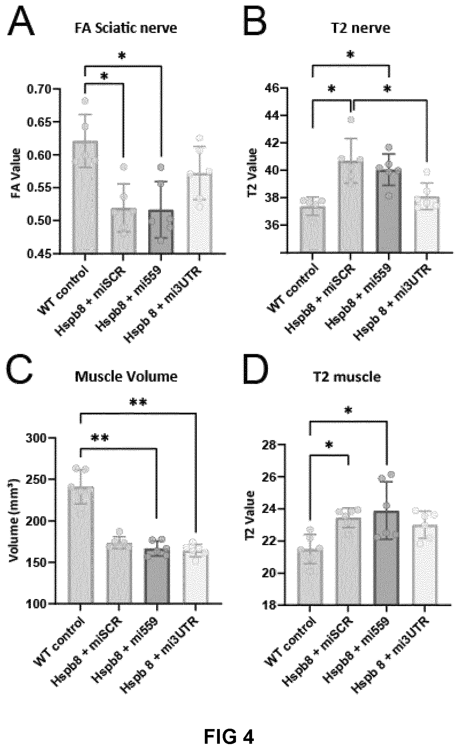

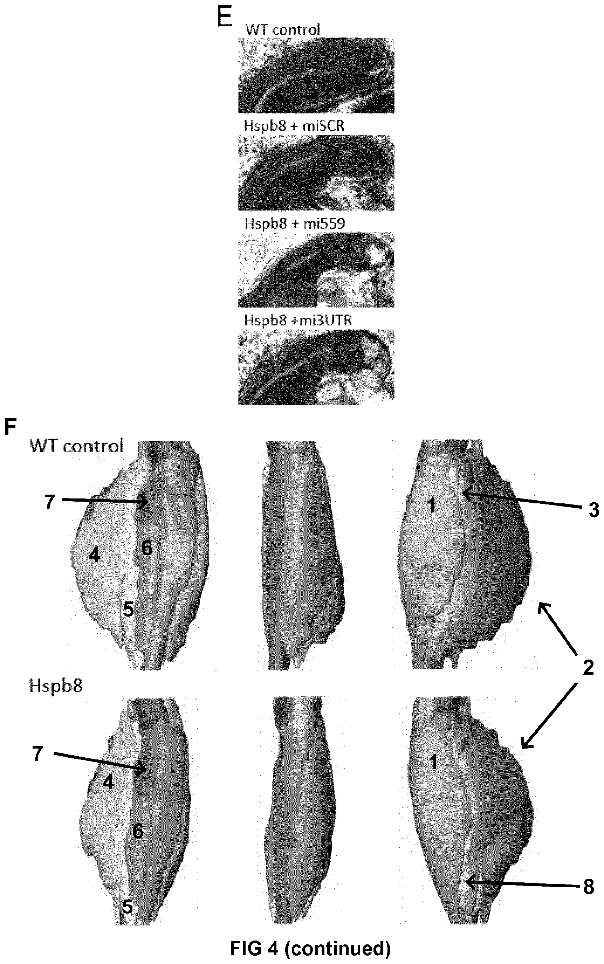

**Figure 4. Specific 3UTR Hspb8 knock-down *in vivo* holds promise for amelioration of sciatic nerve integrity, but fails to rescue muscle atrophy upon MRI analysis. A)** Bar plot of the FA value of sciatic nerve. Each data point is the average FA value of the sciatic nerve in each animal. Error bars are standard deviation. Statistical significance was calculated using Kruskal-Wallis with Dunnetts multiple comparison test (*P < 0.05, **P < 0.01). **B)** $T_2$ value of the sciatic nerve. Each data point is the average $T_2$ value of the sciatic nerve. **C)** Total volume of the different calf muscles. Each data point is the total volume of all calf muscles as displayed in (E). **D)** $T_2$ value of the tibialis anterior muscle. **E)** Representative DW images showing the manual segmentation of the sciatic nerve for each experimental group. **F)** Representative example of 3D reconstruction of calf muscles of an untreated wild type C57bl/6 control and untreated Hspb8 knock-in mouse. This representation includes a lateral, frontal and medial view. Bones are rendered as grey. Muscles are coloured, (1) or teal: *m. tibialis anterior,* (2) or dark purple: *m. lateral medial gastrocnemius,* (3) or light purple: *m. peroneus longus,* (4) or light green: *m. medial gastrocnemius,* (5) or yellow: *m. flexor digitorum longus,* (6) or red: *m. tibialis posterior,* (7) or dark blue: *m. popliteus,* (8) or orange: *m. extensor digitorum longus.* Data are based on the musculoskeletal geometry defined by (Charles et al. 2016). WT Control = untreated wild type C57bl/6 mice, Hspb8 = untreated knock-in mice, Hspb8 + miSCR, Hspb8 + mi559, Hspb8 + mi3UTR treated knock-in mice.

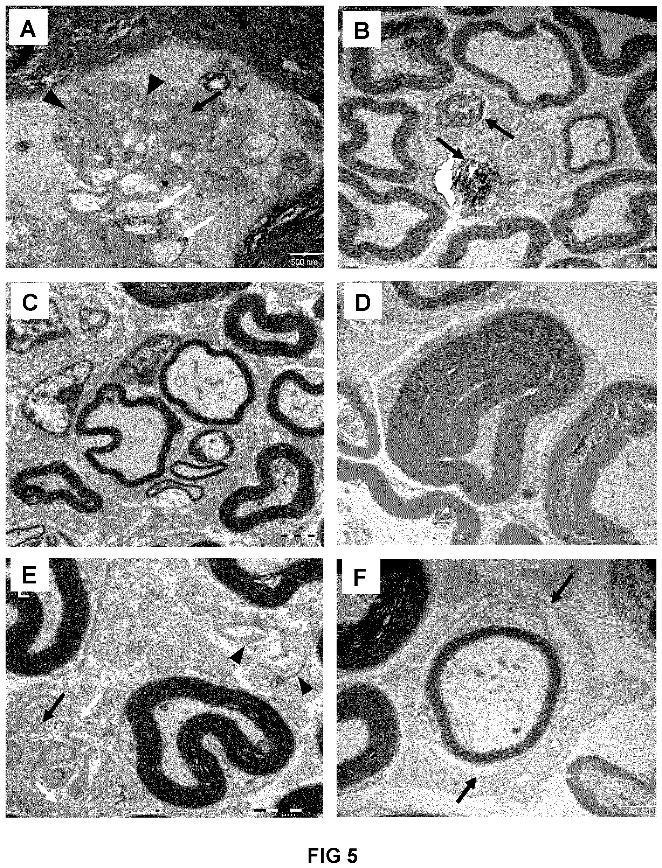

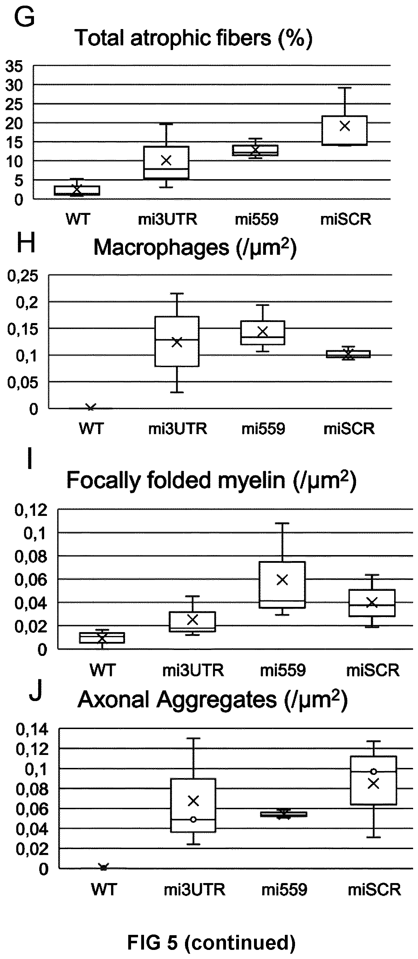

**Figure 5: Quantitative analysis of the muscle-nerve pathology of shRNA-AAV9 treated Hspb8 knock-in mice revealed similar histopathological features as untreated animals.** TEM images illustrate the pathology of mouse sciatic nerve of treated Hspb8 knock-in mice at end-point of 12 months of age and are similar as previously described in untreated mice (Bouhy et al. 2018). **A)** Sciatic nerve axons frequently showed osmiophilic intra-axonal aggregates, composed of amorphous and granular material (arrowheads), small vesicular component resembling smooth ER (black arrow) and larger vacuoles as well as abnormal, enlarged mitochondria (white arrows) some of which undergo mitophagy. **B)** Myelinophage containing large amounts of myelin debris (arrows). **C)** Cluster of regenerating fibers of various axonal diameter with disproportionately thin myelin sheaths. **D)** Abnormal foldings of myelin of a presumably degenerating axon. **E)** Occasionally signs of loss of non-myelinated nerve fibers were observed in the form of collagen pockets (black arrow), remnants of basal lamina (white arrows) and elongated Schwann cell processes without axons (arrowheads). **F)** Axon with disproportionally thin myelin sheath and redundant basal lamina (arrows), indicating remyelination following a demyelinating process. Graphs represent quantification of; **G)** atrophic muscle fibers, **H)** macrophages, **I)** myelin foldings and **J)** intra-axonal aggregates, performed on semithin sections of epon embedded sciatic nerve and gastrocnemius muscle tissue of AVV treated (miSCR, mi559, mi3UTR) homozygous Hspb8$^{K141N/K141N}$ knock-in mice and wild type (WT) mice. Pooled data are from individual animals (n=3) for each of the four groups (Mean+SD).

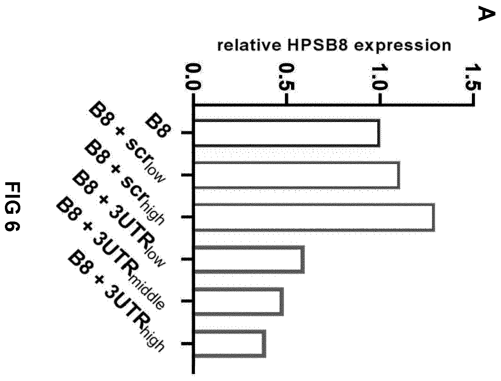

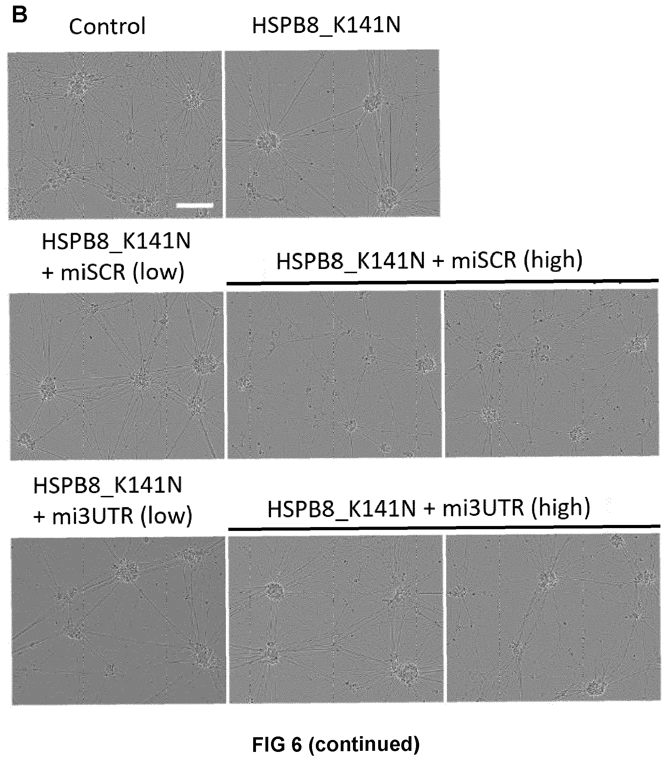

**Figure 6. High viral titters cause neuronal toxicity. A)** RT-qPCR showing the relative expression of HSPB8 at day 24 in human iPSC-derived motor neurons left untreated or transduced with a shRNA construct. "Low" represents the viral titter used for all experiments in Figure 2; "middle" and "high" wells received 2 or 4 times the titter of the low wells, respectively. Data represents one differentiation. B) Phase contrast images taken with the Incucyte S3 Live-Cell Analysis System (Sartorius) at day 24 to visualize the neurite network. "Low" represents the viral titter used for all experiments in Figure 2; "high" wells received 4 times the titter of the low wells. Scale bar = 200 $\mu$m; 10X magnification.

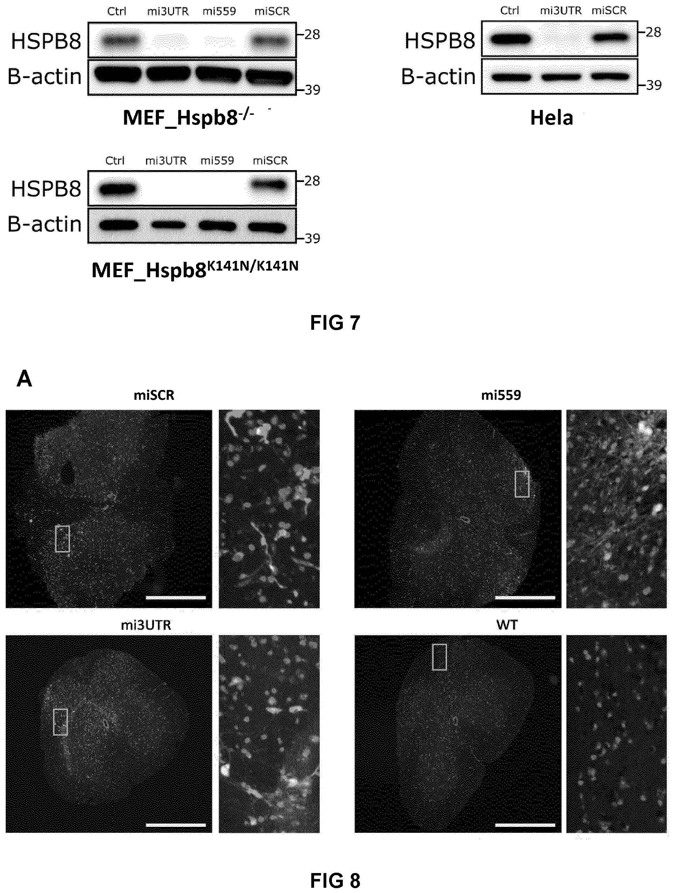

**Figure 7. miRNA-based constructs remain highly efficient in HSPB8/Hspb8 knock-down.** Western blot analysis of stable cell lines expressing miRNAs against human HSPB8 and/or mouse Hspb8. MEF = mouse embryonal fibroblast. Ctrl = untreated wild type C57bl/6 mice, AAV9 treated mice with respectively mi3UTR, mi559 and miSCR shRNA constructs. Molecular weight in kDa.

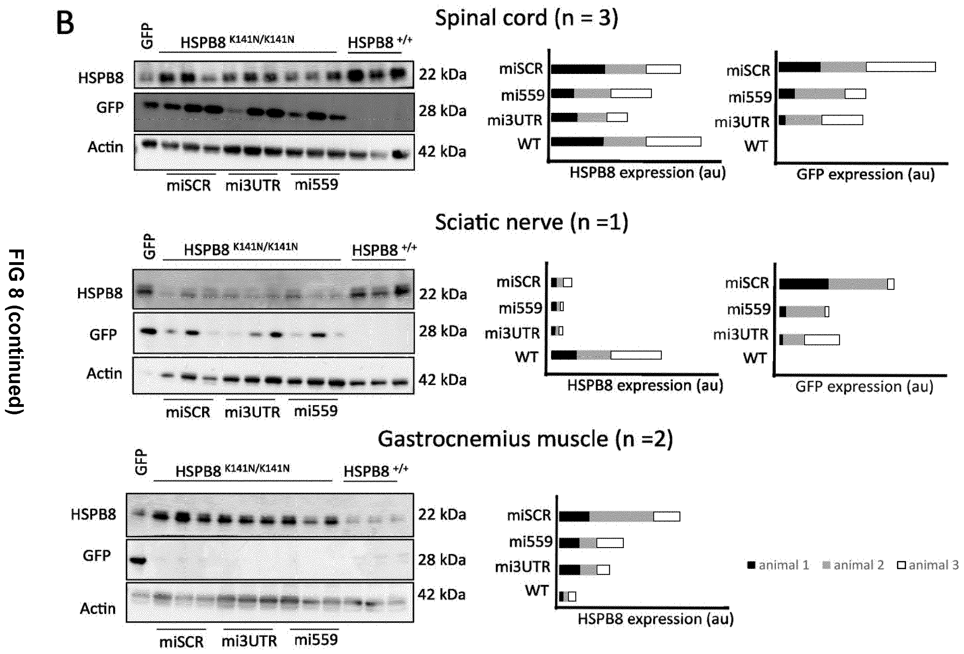

**Figure 8. Immunohistochemistry and western blot analysis to quantify the intrathecal injection of the AAV9 miRNA constructs in the Hspb8 knock-in mouse model.** Intrathecal AAV injection and long-term expression of the GFP-miRNA constructs was verified as end-point experiment on tissues from 12-month-old treated HspB8 knock-in animals next to untreated healthy controls (Hspb8$^{+/+}$) and an LC3-GFP transgenic mouse as positive control (marked as 'GFP'). **A)** Immunohistochemistry on spinal cord sections with anti-GFP (green). Nuclei (magenta) were detected with Hoechst 33342. Rectangular regions (orange) on the overview image indicate the position of the zoom image. Scale bar = 500$\mu$m. **B)** Western blot analysis to quantify GFP and HSPB8 expression in 3 different tissues (lumbar part of the spinal cord, sciatic nerve and gastrocnemius muscle). The treatment (scrambled - SCR, mi3UTR and mi559 miRNA's) is shown by group of 3 animals (dark-grey, grey, light-grey bars represent data from individual animals). Animals were identified by cage number and ear punch (Left/Right marked ear). A representative western blot is shown with quantification for HspB8 and GFP expression (n = number of blots quantified). No GFP signal is detected in muscle.

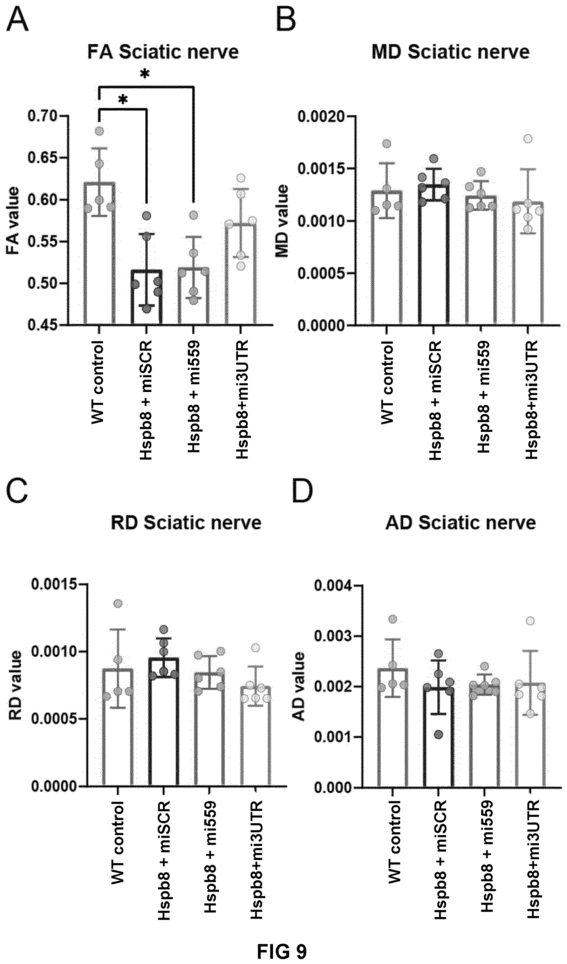

**Figure 9. Overview of DTI parameters extracted from the sciatic nerve.** Statistical analysis to reveal treatment differences in the different diffusion parameters extracted from the sciatic nerve. **A)** Fractional anisotropy (FA), **B)** mean diffusivity (MD), **C)** radial diffusivity (RD), **D)** axial diffusivity (AD). WT control = untreated wild type C57bl/6 mice, Hspb8+miSCR/mi559/mi3UTR treated knock-in mice.

**Overview of sequences**

| 1 | 3' UTR region of human HSPB8 transcript (NCBI Reference Sequence NM_014365.3) | gatgccagtactggcccatccttgttttgtccccaaccctagggcttctctgattcca ggatacattactttagctgaactcagatttagtgcaagtaaaatgttagagggtgc gggggtgaggactgaccacagattccctggatagtgtagtggtagatttctccac aggatagcgcaattggcaaatcatgcttggttgtgttaggccaaaatactagtttt gctttctttacctttctatcttgatgaaaatgttgcacattctatagttgcaaaacaca taaaaggggacttaacatttcacgttgtatcttacttgcagtgaatgcaagggtta cttttctctgggggacctcccccatcacccaggttcctactctgggctcccgattccc atggctcccaaaccatgccgcatggtttggttaatgaaacccagtagctaaccc cactgtgcttccacatgcctggcctaaaatgggtgatatacaggtcttatatcccc atatggaatttatccatcaaccacataaaaacaaacagtgccttctgccctctgc ccagatgtgtccagcacgttctcaaagtttccacattagcactccctaaggacgc tgggagcctgtcagtttatgatctgacctaggtcccccctttcttctgtcccctgtgttt aagtcgggattttacagagggagctgtctccagacagctccatcaggaacca agcaaaggccagatagcctgacagataggctagtggtattgtgtatatgggcg ggacgtgtgtgtcattattatttgagttatgctgttgtttaggggtaaataacagtaa ataattaataataataataataataataaaggagctgacgttctta |
| 2 | Preferred target region in the 3' UTR region of human HSPB8 transcript (human/mouse) | AGGAACCAAGCAAAGGCCAG |
| 3 | Preferred target region in the 3' UTR region of human HSPB8 transcript (mouse) | AAGGAACCAAGCAAAGGCCAG |
| 4 | shRNA: vector DNA sequence | AAGGAACCAAGCAAAGGCCAG CTCGAG CTGGCCTTTGCTTGGTTCCTT |
| 5 | shRNA sequence (with optional overhang) | AAGGAACCAAGCAAAGGCCAG CUCGAG CUGGCCUUUGCUUGGUUCCUU (UU) |
| 6 | shRNA_5UTR target sequence | (AA)TCTGTCTCTCTGAGCCTCT |
| 7 | shRNA_ex1 target sequence | (AA)GATGGCTTTGGCATGGACC |
| 8 | shRNA_3UTR target sequence | (A)AGGAACCAAGCAAAGGCCAG |
| 9 | TRCN0000088558 target sequence | GCTTGGTTGATGGGACTCATT |
| 10 | TRCN0000088559 target sequence | CCACTGTATTTGCCTCGCTTT |
| 11 | TRCN0000088560 target sequence | GCCTTGGAAAGTGTGTGTCAA |
| 12 | TRCN0000088562 target sequence | GTGGAAGTTTCAGGCAAACAT |
| 13 | Scramble_559 target sequence | ATCTCTATCGCCTCGTCTTGT |
| 14 | Mismatch_559 target sequence | CCACCGTACTTGCCTAGCATT |
| 15 | HSPB8 forward primer sequence | CAGAGGAGTTGATGGTGAAGACC |
| 16 | HSPB8 reverse primer sequence | ACTGTCACAGGATCCACCTCTG |
| 17 | HPRT 1 forward primer sequence | TGACACTGGCAAAACAATGCA |
| 18 | HPRT 1 reverse primer sequence | GGTCCTTTTCACCAGCAAGCT |
| 19 | GAPDH forward primer sequence | TGCACCACCAACTGCTTAGC |
| 20 | GAPDH reverse primer sequence | GGCATGGACTGTGGTCATGAG |

(continued)

| | | |
|---|---|---|
| 21 | 3' UTR region of mouse HSPB8 transcript (NCBI Reference Sequence NM_030704.3) | GACGTCAGCCTTGGTCCTTCTTCGCTCCCATCCCCAG CCCCAGGGACTCTCTGATTTGAGGGTACACTACTTTA GCAGCACTCAGATTTAAGGCAACTCAGATGTTGGGGG ATGGGAGGGAGAACCGAACGACCGTCCCTGGAT GATGTAGTAATAGATTTCTCCACAGGGTGACGCAATGG GTCAGCCTTGCTTGGTTGCGTTAGGTCAAAGG ATTGGGAGGTTTTTCTTCATCTTATCTGGGTGAAGACG TCTCGCAGTCTACAGTTGCAAAACAAGCAAAT GGGGACTGAAGATTTCATGTTAAATTTTTACCTTGCAGT TAATGCAAGAGTTGCTTTTCTCTGGGGACCT TCCCATCACCCAGATCCCTGCTCTGGACCCTCCTTCTA CGTGGCTTGGTTGATGGGACTCATTGGCTCAC CTCAGTGTGTTTCTAAACTCTTTGTCTAGAAGAGGTTAT GGGCAGGTCTTATATCCCCATATGGGATTTA TCCTTCACCACACAACACAGTGCCTTTTTTTTTGCAGCAT GACCCCATGGCTCTCAAACCATCACCACCAA GAACTCTGGGAGCCCCTCAGTTAATTCTCTGGCTAAGG TCCCCCTTTATTGTGTCCCCTGTGTCCAAGTC ACATTTTTACAGAGAGCTGTCTTGGAGCAGCTCCGCAA GGAACCAAGCAAAGGCCAGACAGCCTGCACGC AGGCTAGTGGCATTGTGTGTGTGTTTGAGGGGTGGGG AGGATGTGTGTGTCTTTGTTGTGTGAATTGTGC TGTTGTTTAGAGGGAAAATAACGGTGAATAATAATGAT GATAATAAAGGAGCTGATGTTCTTAGCAAAAA AAAAA |
| 22 | Human HSPB8 (NCBI Reference Sequence: NP_055180.1) | MADGQMPFSCHYPSRLRRDPFRDSPLSSRLLDDGFGMDP FPDDLTASWPDWALPRLSSAWPGTLRSGMVP RGPTATARFGVPAEGRTPPPFPGEPWKVCVNVHSFKPEE LMVKTKDGYVEVSGKHEEKQQEGGIVSKNFT KKIQLPAEVDPVTVFASLSPEGLLIIEAPQVPPYSTFGESSF NNELPQDSQEVTCT |
| 23 | Human synapsin I promoter | ctgcgctctc aggcacgaca cgactcctcc gctgcccacc gcagactgag gcagcgctga<br>gtcgccggcg ccgcagcgca gatggtcgcg cccgtgcccc cctatctcgc gcctcgcgtg<br>gtgcggtccg gctgggccgg cggcggcgcg gacgcgacca aggtggccgg gaaggggagt<br>ttgcgggga ccggcgagtg acgtcagcgc gccttcagtg ctgaggcggc ggtggcgcgc<br>gccgccaggc gggggcgaag gcactgtccg cggtgctgaa gctggcagtg cgcacgcgcc<br>tcgccgcatc ctgtttcccc tccccctctc tgatagggga tgcgcaattt ggggaatggg<br>ggttgggtgc ttgtccagtg ggtcggggtc ggtcgtcagg taggcacccc caccccgcct<br>catcctggtc ctaaaaccca cttgcact |

(continued)

| 24 | CMV enhancer | GGCATTGATTATTGACTAGTTATTAATAGTAATCAATTACGGGGTCATTAGTTCATAGCCCATATATGGAGTTCCGCGTTACATAACTTACGGTAAATGGCCCGCCTGGCTGACCGCCCAACGACCCCCGCCCATTGACGTCAATAATGACGTATGTTCCCATAGTAACGCCAATAGGGACTTTCCATTGACGTCAATGGGTGGAGTATTTACGGTAAACTGCCCACTTGGCAGTACATCAAGTGTATCATATGCCAAGTCCGCCCCTATTGACGTCAATGACGGTAAATGGCCCGCCTGGCATTATGCCCAGTACATGACCTTACGGGACTTTCCTACTTGGCAGTACATCTACGTATTAGTCATCGCTATTACCATG |

## Examples

Material and Methods

*Ethics statement*

All experimental procedures were in agreement with the European directives, Belgian and Flemish laws and were approved by the Ethics Committee on Animal care and use of the University of Antwerp, Belgium (2020-66). Mice were housed under the care of the Animal Facility Interfaculty Unit, which is accredited by the Association for Assessment and Accreditation of Laboratory Animals. All experiments were performed on adult female Hspb8$^{+/+}$ (wild type, WT) and Hspb8$^{K141N/K141N}$ (knock-in, KI) mice, which have been described in detail (Bouhy et al. 2018). All experiments performed using patient material for iPSC experiments were approved by the Committee for Medical Ethics (University of Antwerp).

*Plasmids*

The pLKO.1 - TRC cloning vector was obtained from Addgene (#10878). Design of short-hairpin RNA (shRNA) oligo's for targeting both human HSPB8 and mouse Hspb8 was performed according to the guidelines in the Addgene protocol (https://www.addgene.org/protocols/plko/). Oligo's were ordered from IDT (Integrated DNA Technologies, Leuven, Belgium) and cloned into the pLKO.1 vector following the Addgene protocol. Mouse specific shRNAs of the Sigma Mission pLKO.1-library were obtained from the BCCM/GeneCorner Plasmid Collection (https://bccm.belspo.be/). Four shRNAs against murine Hspb8 (TRCN0000088558, TRCN0000088559, TRCN0000088560 and TRCN0000088562) were used in this study. As controls, we generated a scrambled (https://www.genscript.com/tools/create-scrambled-sequence) and a mismatch (three mismatches, of which two are located in the seed region) version of the TRCN0000088559 shRNA. The shRNA-targeting sequences are provided in Table 1.

Table 1. shRNA target sequences and additional information

| Name | Short name | Target sequence | Species | Region |
|---|---|---|---|---|
| shRNA_5UTR | mi5UTR | (AA)TCTGTCTCTCTGAGCCTCT SEQ ID NO: 6 | Mouse / Human | 5'-UTR |
| shRNA_ex1 | miEx1 | (AA)GATGGCTTTGGCATGGACC SEQ ID NO: 7 | Mouse / Human | CDS |
| shRNA_3UTR | mi3UTR | (A)AGGAACCAAGCAAAGGCCAG SEQ ID NO: 8 | Mouse / Human | 3'-UTR |
| TRCN0000088558 | mi558 | GCTTGGTTGATGGGACTCATT | Mouse | 3'-UTR |
| | | SEQ ID NO: 9 | | |
| TRCN0000088559 | mi559 | CCACTGTATTTGCCTCGCTTT SEQ ID NO: 10 | Mouse | CDS |
| TRCN0000088560 | mi560 | GCCTTGGAAAGTGTGTGTCAA SEQ ID NO: 11 | Mouse | CDS |
| TRCN0000088562 | mi562 | GTGGAAGTTTCAGGCAAACAT SEQ ID NO: 12 | Mouse | CDS |
| Scramble_559 | SCR | ATCTCTATCGCCTCGTCTTGT SEQ ID NO: 13 | - | - |

(continued)

| Name | Short name | Target sequence | Species | Region |
|------|-----------|-----------------|---------|--------|
| Mismatch_559 | MM | CCACCGTACTTGCCTAGCATT SEQ ID NO: 14 | - | - |

Legend: UTR: untranslated region, CDS: coding sequence, mi: micro-RNA. (A) or (AA) are mouse specific Adenine bases.

*Cell culture*

All cell lines (HeLa, HEK293T, NSC34) were acquired from the American Type Culture Collection (ATCC). The HeLa cells (derived from human cervical cancer cells) were grown in MEM medium supplemented with 10% (v/v) foetal bovine serum (FBS) and 1% (v/v) Penicillin-Streptomycin (P/S) (Life Technologies, Carlsbad, CA, USA). The HEK293T (derived from human embryonal kidney) and NSC34 (mouse motor neuron-like cell line) cells were grown in DMEM medium supplemented with 10% (v/v) FBS and 1% (v/v) P/S (Life Technologies, Carlsbad, CA, USA). All cells were cultured at 37°C and 5% $CO_2$ in a cell incubator (Steri-Cycle i160, Thermo Scientific, Merelbeke, Belgium).

Mouse embryonic fibroblasts (MEFs) from our in-house Hspb8 mouse models (Bouhy et al. 2018) were isolated according to the protocol published by (Xu 2005). Briefly, breeding pairs were set up and females were checked for copulation plugs the next morning, with plugged females being separated from the male breeder. On embryonic day E13.5, the pregnant female was euthanized with $CO_2$ and the intact uterus was extracted. Embryos were removed from their vitelline membrane and each embryo was separated from its placenta and surrounding membranes. The tail was saved for genotyping. Forceps were used to remove the brain and the dark red organs (liver, heart and umbilical cord) such that the majority of remaining cells were fibroblasts. In a new 2ml Eppendorf tube, the embryo body was minced in small pieces with sterile scissors, in the presence of 0.25% trypsin. The minced tissue was transferred to a 15 ml conical tube, followed by three rounds of 2 min incubation at 37°C - short vortex - pipet up and down. After the last round, complete DMEM (DMEM + 10% FBS + 1% P/S + 1% glutamine) was added and the cell suspension was placed on a 70 $\mu$m cell strainer (431751, Corning) to remove tissue debris, followed by plating in a T25 flask. Primary MEFs (passage 3) were immortalized with the pSV51 plasmid expressing SV40 large T-antigen (Huylebroeck et al. 1988) (https://bccm.belspo.be/; accession number LMBP 1829).

*Generation of stable cells through lentiviral transduction*

Cell lines stably expressing the shRNA constructs were generated by lentiviral transduction. To this end, HEK293T cells were transiently transfected with packaging (pCMV dR8.91), envelope (pMD2-VSV), and pLKO.1 plasmids using polyethylenimine hydrochloride PEI MAX 40K (24765-1, PolySciences Europe, Hirschberg an der Bergstrasse, Germany). After 48 h, the virus containing supernatant was collected from the HEK293T cells, filtered through a 0.45 $\mu$m filter (SLHV033RB, Millipore, Burlington, MA, USA) and used to infect the destination cells. Virus medium was removed 24 h after transduction, followed by selection for infected cells by adding puromycin (1 $\mu$g/ml; ant-pr-1, InvivoGen Europe, Toulouse, France).

*Differentiation of iPSCs into motor neurons*

The generation and maintenance of control and CMT2L iPSC lines was described elsewhere (Van Lent et al. 2021). For differentiation of iPSCs into motor neurons, we slightly adapted a previously published protocol (Guo et al. 2017). Briefly, at the start of the differentiation, Essential 8™ Flex medium was replaced by neuronal medium, consisting of Neurobasal™ Medium (10888022, Thermo Fisher), DMEM/F12 medium (31330038, Thermo Fisher), N2 supplement (17502048, Thermo Fisher), B-27™ supplement (without vitamin A) (12587010, Thermo Fisher), GlutaMAX™ (35050061, Thermo Fisher), β-mercaptoethanol (11528926, Thermo Fisher) and ascorbic acid (A8960-5G, Sigma-Aldrich). During the first 2 days, dual SMAD inhibitor SB431542 (1614/10, Bio-Techne), dual SMAD inhibitor LDN-193189 (QT106329, Selleck Chemicals) and WNT agonist CHIR99021 (4423/10, Bio-Techne) were added to the neuronal medium. On the following days, half of the medium was replaced with media containing compounds necessary for motor neuron differentiation and maturation. Compounds used included: retinoic acid (R2625-50MG, Sigma Aldrich), smoothened agonist (SAG, 4366/1, Bio-Techne), brain derived neurotrophic factor (BDNF, 450-02, Peprotech), glial cell derived neurotrophic factor (GDNF, 450-10, Peprotech), γ-secretase inhibitor DAPT (2634/10, Bio-Techne), ciliary neurotrophic factor (CNTF, 450-13, Peprotech) and laminin (L2020-1MG, Sigma Aldrich). Motor neuron progenitors were subsequently plated on laminin-coated plates. To enhance the purity of the motor neurons, a single treatment with 1 $\mu$M cytosine β-D-arabinofuranoside (Ara-C, C1768, Sigma Aldrich) was performed on Day 14.

*Lentiviral transduction of iPSC-derived motor neurons*

Lentiviral transduction was used to stably express shRNAs in iPSC-derived motor neurons. To this end, HEK293T cells were transiently transfected with packaging (pCMV dR8.91), envelope (pMD2-VSV), and pLKO.1 plasmids using PEI MAX 40K (24765-1, PolySciences Europe, Hirschberg an der Bergstrasse, Germany). After 48 h, the virus containing supernatant was collected from the HEK293T cells, filtered through a 0.45 μm filter (SLHV033RB, Millipore, Burlington, MA, USA) and concentrated using an Amicon Ultra-15 Centrifugal Filter Unit (UFC905024, Millipore, Burlington, MA, USA). Lentiviral titer was quantified using Lenti-X GoStix Plus (631280, Takara Bio, Kusatsu, Shiga, Japan) in order to keep the viral concentration/well constant in all experiments. Motor neurons were transduced at day 14 of differentiation, and virus-containing medium was gradually replaced by differentiation medium from 48 h after transduction onwards.

*Reverse transcriptase quantitative PCR*

Total RNA was extracted using the Universal RNA Purification Kit (E3598, GeneMATRIX) according to manufacturer's instructions. RNA was reverse transcribed into cDNA using M-MLV Reverse Transcriptase (28025013, Thermo Fisher). For each sample, 10 ng cDNA was then amplified using the SYBR Green dye (4368708, Thermo Fisher) on the QuantStudio™ 6 Flex Real-Time PCR System (Thermo Fisher). Relative gene expression was calculated using the ΔΔCT method (Hellemans et al. 2007) and normalized with the geometric mean of internal reference genes (GAPDH, HPRT1) in the qbase+ software (Biogazelle, Zwijnaarde, Belgium). The primer sequences used in this study can be found in Table 2.

Table 2. Primers used for qPCR experiments

| Gene | Forward primer sequence (5'-3') | Reverse primer sequence (5'-3') |
|---|---|---|
| HSPB8 | CAGAGGAGTTGATGGTGAAGACC SEQ ID NO: 15 | ACTGTCACAGGATCCACCTCTG SEQ ID NO: 16 |
| HPRT1 | TGACACTGGCAAAACAATGCA SEQ ID NO: 17 | GGTCCTTTTCACCAGCAAGCT SEQ ID NO: 18 |
| GAPDH | TGCACCACCAACTGCTTAGC SEQ ID NO: 19 | GGCATGGACTGTGGTCATGAG SEQ ID NO: 20 |

*Mitochondrial morphology analysis*

Motor neurons were cultured on 8 well-plates (Ibidi) and incubated with a final concentration of 250 nM MitoTracker™ Red CMH2Xros (Life Technologies) in neuronal medium for 25 minutes at 37°C. Afterwards, the medium was replaced with fresh neuronal medium. Live-cell imaging at 540 nm was performed using a Carl Zeiss Axiovert 200M microscope equipped with an incubation chamber (37°C, 5 % CO2). Images were taken with an AxioCam MR Rev3 camera and 40X objective.

Images were analyzed using the Fiji distribution of imaged (Schindelin et al. 2012; Schneider, Rasband, and Eliceiri 2012). Images were used to measure mitochondrial morphology by detecting individual mitochondria segments as described (Van Lent et al. 2021). Briefly, a segmentation procedure was executed using an imaged macro script that combined global (Median method) and local (Phansalkar method) automatic intensity threshold and Boolean operations on the binary masks. The aspect ratio (termed as the ratio between the major and minor axis) of mitochondrial segments was measured to describe the mitochondrial elongation morphology.

*Recombinant AAV production and purification*

After *in vitro* testing, the target sequence of lead candidate or scramble shRNAs were cloned into a miR30 scaffold (Sun et al. 2006; Osório et al. 2014). Next, the hairpins were subcloned into the AAV2/9 transfer plasmid, downstream of a CMVie-enhanced synapsin promoter and eGFP, resulting in pAAV-miRNA plasmids (pAAV-mi3UTR, pAAV-mi559 and pAAV-miSCR). Viral vectors were produced by the Leuven Viral Vector Core (LVVC; https://gbiomed. kuleuven.be/english/corefacilities/LVVC/). Vector production and purification was performed as previously described (Van der Perren et al. 2011). Briefly, HEK293T were seeded in a Hyperflask Cell Culture Vessel (1720 cm$^2$ growth area, Corning Life Sciences, Kennebunk, ME, USA). The next day, cells were transfected with the pAAV-miRNA, AAV rep/cap and pAdbDeltaF6 plasmids in the ratio of 1:1:1 using a 25-kDa linear polyethylenimine solution. The supernatant was harvested 3 days after transfection and concentrated using tangential flow filtration. The concentrated supernatant was purified using an iodixanol step gradient, aliquotated and stored at-80 °C. Real-time PCR analysis was used for vector genome copy determination. All vectors and corresponding titers are listed in Table 3.

Table 3. AAV9 vectors used in this study

| Short name | Description | Titer (GC/ml) |
|---|---|---|
| AAV9.mi559 | 21-008_AAV2/9_CMVie synapsin-intron-eGFP-1x miR HSPB8 559 | 1.12E+12 |
| AAV9.mi3UTR | 21-009_AAV2/9_CMVie synapsin-intron-eGFP-1x miR HSPB8 3UTR | 1.25E+12 |
| AAV9.miSCR | 21-010_AAV2/9_CMVie synapsin-intron-eGFP-1x miR HSPB8 SCR | 7.76E+11 |

*Intrathecal AAV vector injection*

After anesthetizing the animals with 5% isoflurane, we delivered the AAV2/9 vectors via a single intrathecal injection as previously described (Hylden and Wilcox 1980). Briefly, following a small skin incision along the lower lumbar spine level to visualize the spine, the viral particles were delivered into the L5-L6 intervertebral space. A 50-μL Hamilton syringe (HA80530, Filter Service, Eupen, Belgium) connected to HAMILTON RN-needle (7758-02, Filter Service, Eupen, Belgium) was used to inject 10 μl of AAV stock containing ~0.8-1.2 × $10^{10}$ genome copies (gc)/ml. Each vector was injected slowly at a rate of 5μl/min and the needle left in place for 5-10 seconds before withdrawal. A flick of the tail was considered indicative of correct positioning of the needle. Vetbond Tissue Adhesive (3M) was used to close the small incision wound. Each animal was allowed to wake up and monitored until ambulatory. Throughout the whole procedure, animals were kept in a warm environment until they recovered from anaesthesia.

*Behavioral analysis - Rotarod*

General motor performance was assessed using a five lane Rota-Rod Treadmill for mouse (ENV-574M, Med associates Inc., St Albans, VT, USA). Animals were trained for five consecutive days before each test. Each animal was given three trials on an accelerating rotating rod (4-40 rpm on 5 min ramp duration), with a 1 min resting interval in between. The latency to fall was recorded and the longest time spent on the rotarod was used as the final measure of motor performance.

*Electrophysiological analysis - nerve conduction studies*

Nerve conduction studies were performed using the Neuro-EMG-Micro system (Neurosoft, Ivanovo, Russia) as previously described (Bouhy et al. 2018; Pollari et al. 2018). Briefly, subdermal 0.4-mm electrodes were used for stimulation and recording on anesthetized mice (5% isoflurane). Compound muscle action potential (CMAP) amplitudes were measured by placing stimulating electrodes subcutaneously on both sides of the sciatic notch and the recording electrode at the level of the gastrocnemius muscle. All recording were measured at supramaximal stimulation. Three consecutive recordings were performed, with the highest recorded amplitude and the associated latency being used as final scores.

*MRI data acquisition*

All MRI measurements were performed on a 7T horizontal MR system (Pharmascan 70/16 US, Bruker Biospin, Germany) using the cross-coil setup with a 72 mm diameter quadrature volume coil and a 20 mm 4-channel array surface coil for mice (Bruker Biospin, Germany) at the Bio-Imaging Lab of the University of Antwerp. Of each experimental group [untreated healthy controls (WT) and Hspb8 knock-in mice treated with miSCR, mi3UTR and mi559], 6 mice were included in the MRI experiment. The mice were anesthetized using an induction dose of 5% isoflurane (Isoflo®, Abbot Laboratories Ltd.) with a mixture of oxygen and nitrogen at flow rates of 100 and 200 cm$^3$/min respectively. After positioning the mouse with the left leg on top of the surface coil, the anaesthetic dose was lowered to 2% isoflurane to maintain anaesthesia. A small pneumatic sensor (SA Instruments, NY, USA) and temperature probe were positioned under the animal to monitor the breathing rate and body temperature respectively. The body temperature was maintained constant using a warm air system with feedback unit (SA Instruments, NY, USA).

Each imaging session started with short $T_2$ weighted anatomical scans (TE 33ms, TR 2500, RARE factor 8) to ensure a proper positioning of the leg within the scanner. First, we acquired images at the level of the calf muscle. A field map was acquired to perform second-order iterative shimming on the calf muscle, to ensure homogenous signal from this region. Next, a $T_1$-weighted multislice FLASH (Fast Low-Angle Shot) image of the calf muscle was acquired (TE 3.5 ms, TR 500 ms, 3 averages, flip angle 30°) with a high in-plane resolution (pixel size 0.052 × 0.052 mm$^2$, field of view (FOV) 20 × 20 mm, matrix size 384 × 384, slice thickness 0.4 mm, slice gap 0.1 mm, 29 axial slices acquired in left-right read direction) to determine the calf muscle volume. Fat suppression was achieved by a hermite pulse of 5.2ms/1038 Hz duration/-bandwidth followed by a 2-ms-long gradient spoiler. In addition, a $T_1$-weighted FLASH image was acquired without fat suppression, while the other acquisition parameters remained the same.

Subsequently, multislice multiecho (MSME) $T_2$ mapping of calf muscle was acquired: TR 6000 ms, TE in multiples of 7 ms ranging from 7 to 140 ms (20 values). The calf muscle was sampled with 14 slices with the following anatomical parameters were: FOV $20 \times 20$ mm$^2$, matrix $128 \times 128$, pixel size $0.156 \times 0.156$, slice thickness 0.7 mm, slice gap 0.05 mm. Fat suppression as described above in the FLASH sequence.

In a second phase of the imaging session, the focus shifted towards the sciatic nerve positioned parallel to the femur. Therefore, we repeated the fieldmap and second-order iterative shimming, but this time with a focus on the hind leg. Anatomical $T_2$-weighted scans (TE 33ms, TR 2500, RARE factor 8) were performed to determine a slice position where most of the sciatic nerve would be covered by one slide. In this specific location, a MSME $T_2$ map and diffusion weighted image (DWI) were acquired. The MSME T2 maps was acquired with the same acquisition parameters as calf muscle $T_2$ map, but using only 3 slices instead of 14. DWI were acquired using 4 shot spin-echo echo-planar imaging (SE-EPI) with the following acquisition parameters: TE 17.5 ms, TR 2500 ms, $\delta$ 2.5ms, $\Delta$ 8.4 ms, b-value 670 s/mm$^2$, six diffusion gradient directions and one b0 image. The anatomical parameters were the same as for the $T_2$ maps of the sciatic nerve.

Total acquisition time of all MRI scans amounted to approximately 2 hours. After the scanning session, the mice were left to recover in a warmed recovery box before returning to their home cage.

*MRI data processing*

We used Amira 4.5 (Mercury Computer system Inc., San Diego, CA) to delineate the different muscles in the calf following the musculoskeletal geometry described in Charles et al. (2016) (Charles et al. 2016). The $T_2$ maps were processed by fitting the signal decay for each voxel using the following formula:

$$S(t) = bias + S(0)e^{\frac{-t}{T_2}}$$

Where '$t$' is the echo time of acquisition, '$S(t)$' the measured signal at time t. The parameter fitting of parameter *bias, S(0)* and $T_2$ was done using an in-house developed MATLAB tool by the Vision Lab. We delineated the tibialis anterior muscle for the T2 analysis to assess muscle integrity.

Diffusion tensor imaging (DTI) parameters were calculated from the DWI using Mrtrix3 version 3.0 (Tournier et al. 2019) after correcting for noise, and Gibb's ringing artefacts. Specific regions of interest were delineated in each of the individual scans blind to the treatment conditions.

*Tissue harvesting and processing for western blotting and electron microscopy*

For the end-point experiment; after inhalation of a lethal dose of $CO_2$, the sciatic nerve, gastrocnemius muscle left side and spinal cord were dissected from half the animals; snap frozen in liquid nitrogen, and stored at -80 °C for subsequent protein analysis. The sciatic nerve, gastrocnemius muscle of the right side, were processed for electron microscopy. From the other half of the animals, spinal cord was flushed and processed for immunohistochemistry.

*Western blot*

Cells were collected by centrifugation and lysed in RIPA lysis buffer (1% Trition X-100, 150 mM NaCl, 0.1% SDS, 0.5% deoxycholic acid, 50 mM Tris.HCl pH 7.5) supplemented with Halt™ Protease Inhibitor Cocktail (78429, Life Technologies, Carlsbad, CA, USA) and PhosSTOP™ (4906837001, Roche Applied Science, Penzberg, Germany) for 15 min on ice and cleared by centrifugation. Tissue samples were homogenized in complete RIPA lysis buffer using a tissue grinder (357844, Wheaton), followed by a 20 min incubation on ice, sonication and clearing by centrifugation. Protein concentrations were determined with the Pierce BCA assay (23225, Thermo Fisher Scientific, Waltham, MA, USA). Equal amounts of protein lysate were mixed with reducing NuPAGE LDS Sample buffer (NP0007, Life Technologies, Carlsbad, CA, USA) supplemented with 100 mM 1,4-dithiothreitol (DTT). Samples were boiled at 95°C for 5 min, before loading on a 4-12% SDS-PAGE gel (NP0322BOX, Life Technologies, Carlsbad, CA, USA) with SeeBlue Plus2 Pre-stained Protein Standard (LC5925, Life Technologies, Carlsbad, CA, USA). After separation, proteins were transferred to a nitrocellulose membrane (RPN132D, GE Healthcare Life Sciences, Amersham, UK). Depending on the antibody used, membranes were blocked with 5% not-fat milk in PBS-Tween or with 5% BSA in TBS-Tween. Afterwards, membranes were incubated with primary antibodies for 1 h at room temperature or overnight at 4°C, followed by incubation with horseradish peroxidase (HRP)-conjugated secondary antibodies. Blots were developed by using either Pierce™ ECL Plus Western Blotting Substrate (32132, Life Technologies, Carlsbad, CA, USA) or SuperSignal™ West Femto Maximum Sensitivity Substrate (34096, Thermo Fisher Scientific, Waltham, MA, USA) and imaged with an Amersham Imager 600 (GE Healthcare, Wauwatosa, WI, USA). All antibodies used in western blotting are listed in Table 4.

Table 4. Antibodies used in this study

| Abbreviation | Antigen | Host species | Dilution | Source |
|---|---|---|---|---|
| HSPB8 | Heat Shock Protein Family B (Small) Member 8 | Rabbit polyclonal | 1:500 | Cell Signaling, #3059 |
| BAG3 | Bcl2-associated athanogene 3 | Rabbit polyclonal | 1:1000 | Proteintech, 10599-1-AP |
| β-actin | Beta-actin | Mouse monoclonal | 1 :5000 | Sigma-Aldrich, A5441 |
| GFP | Green fluorescent protein | Rabbit polyclonal | 1:1000 | Abcam, ab290 |

*Immunohistochemistry of the spinal cord*

After dissection by flushing the spinal cord, the lumbar part was rinsed in PBS and fixed in 4% paraformaldehyde solution overnight at room temperature. After fixation, the tissue was washed with sucrose solution, containing 130 mM $Na_2HPO_4$, 30 mM $KH_2PO_4$, 10% (w/v) sucrose, 0.01% sodium azide and 0.03% Bacitracin, pH 7.2, overnight at room temperature. After washing with the sucrose buffer, the tissue was mounted in OCT embedding compound, and frozen at -20 to -80 °C. Using a cryostat, 7 $\mu$m thick tissue sections were cut and thaw-mounted onto gelatine-coated histological slides. The slides were dried for 30 minutes on a slide warmer at 37 °C, then frozen at -80 °C. Starting the fluorescent staining, the slides were thawed and air dried at room temperature for 20 minutes. A barrier pen was used to surround the tissue with a hydrophobic barrier. The slides were rehydrated in PBS 0.137 M NaCl, 0.05 M $NaH_2PO_4$, pH 7.4) for 10 minutes. Permeabilization was done in 0,5% Tween-20 in PBS for 30 minutes. Non-specific staining was blocked, by incubating in blocking buffer containing, 1% BSA, 5% normal goat serum (005-000-121, Bio-Connect B.V., Huissen, The Netherlands) and 0.05% Tween 20 in PBS, for 60 minutes at room temperature. Slides were incubated with GFP-Booster_Atto488 (gba488-100, ChromoTek GmbH, Planegg, Germany) diluted 200x in blocking buffer for 60 minutes at room temperature. After incubation, slides were washed with PBS, then incubated with Hoechst 33342 10mg/ml (Life Technologies H3570) 5000x diluted in PBS for 3 minutes. After a last wash with PBS, coverslips were mounted with an anti-fade mounting medium (S302380-2, Dako Belgium NV/Agilent Technologies, Zaventem, Belgium). Samples were imaged on a Nikon Ti2 microscope controlled by NIS Elements AR (version 5.42.01) and equipped with a Photometrix Kinetix sCMOS camera, and using a 10x $\lambda$D PlanApo objective (NA 0.45) (Nikon, Groot-Bijgaarden, Belgium). Tile scanning (2x2) was used with overlapping 2720 $\times$ 2720 images (lateral pixel dimension 0.65 $\mu$m) to image entire sections (resulting in images of up to 3.3mm $\times$ 3.3 mm).

*Histopathology*

Muscle and nerve pathologies were performed on 12 months old mice, age at which the disease shows complete penetrance. Muscle and nerve (*M. gastrocnemius* and *N. sciaticus*) tissue from untreated healthy control mice and treated Hspb8 homozygous knock-in mice were harvested and fixed in 3.9% glutaraldehyde. The fixed tissue samples were processed for epon-embedding for semithin-section light microscopy and ultrathin section electron microscopy analysis as described previously (Groh et al. 2012; Bouhy et al. 2018). Images of the semithin-section were taken with a Zeiss Axiocam and were quantified with the imaged software.

*Statistics*

All statistical tests were performed using the GraphPad Prism software (version 9.3.1). Data containing more than two groups were analysed with one-way ANOVA followed by Dunnett's multiple comparisons test. Due to the low sample size, statistical analysis of the MRI data was performed with non-parametric alternative of the one-way ANOVA, the Kruskal-Wallis test. To compare treatment groups over time, a 2-way ANOVA with Dunnetts's multiple comparison test was used. $*P < 0.05$, $**P < 0.01$, $***P < 0.001$, $****P < 0.0001$ were considered significant. Data values represent the mean $\pm$ standard error of the mean (SEM), except otherwise mentioned.

Example 1: Designing and validating micro-RNAs to suppress HSPB8/Hspb8 expression in human and mouse

To test whether knock-down of HSPB8 using RNA interference would be effective, we designed three short-hairpin RNAs (shRNAs) with a guide strand specifically targeting both human *HSPB8* and mouse *Hspb8* mRNA for further degradation **(Figure 1A).** Although human HSPB8 (UniProtKB accession number Q9UJY1) and mouse Hspb8 (Uni-ProtKB accession number Q9JK92) are rather similar at the protein level (94.4% identity in 196 amino acid residues overlap), their transcripts are significantly different, making it difficult to target both mRNAs (reference human NM_014365 and mouse MGI:2135756) with one single RNA interference molecule. We therefore included four different mouse-specific shRNAs to increase the chance of finding a potent shRNA that could be used in our mouse model **(Table 1).** To test their potency, we generated different cell lines stably expressing the shRNA constructs and assessed HSPB8 protein levels by immunoblotting. In murine cell lines, all shRNAs - except mi-5UTR and mi-ex1 - did show a significant decrease in Hspb8, with no pronounced difference between Hspb8 wild type (WT) and mutant (K141N) mouse embryonic fibroblasts (MEFs) and mouse motor neuron like NSC34 cells **(Figure 1B).** In HeLa cells, the mi3UTR shRNA was also very effective in reducing human HSPB8 expression, with almost no signal detected on the western blot **(Figure 1C).** Based on these results, we decided to proceed with the mi3UTR (targeting both human HSPB8 and mouse Hspb8) and the mi559 (specific for mouse Hspb8) shRNAs.

Example 2: HSPB8 knock-down in CMT2L patient derived iPSCs ameliorates mitochondrial morphology in motor neurons

Because HSPB8 knock-down could be beneficial in patient-derived cells, we took advantage of an established heterozygous HSPB8_K141 (dHMN/CMT2L) patient-derived iPSC line, that was characterized to elucidate the underlying mechanism of axonal degeneration shared by different CMT2 subtypes (Van Lent et al. 2021). We differentiated iPSCs from the HSPB8_K141N patient towards motor neurons and introduced the shRNAs (miSCR - scrambled, or mi3UTR) via lentiviral transduction at day 14, after which motor neurons were allowed to mature until day 38-42 (Figure 2A). The iPSCs from a healthy unrelated individual were taken along during the differentiation to serve as a control (Van Lent et al. 2021). At the end of the differentiation (day 38-42), a clear reduction in *HSPB8* mRNA levels was observed in mi3UTR (but not in miSCR) shRNA treated cells, thereby confirming the potency of the mi3UTR molecule to reduce HSPB8 expression throughout the differentiation of human derived motor neurons **(Figure 2B).**

The HSPB8_K141N mutation was previously shown to induce mitochondrial aggregation and morphological changes in a cellular model and in a sural nerve biopsy of a dHMN/CMT2L patient, along with increased oxidative stress (Yang et al. 2017). Similar mitochondrial morphology alterations were observed in iPSC-derived motor (but not sensory) neurons of the HSPB8_K141N patient (Van Lent et al. 2021). We analysed the mitochondrial morphology in mature HSPB8_K141N motor neuron cultures labelled with MitoTracker™ dye. The mi3UTR was able to rescue the increased mitochondrial swelling and fragmentation (as shown by a decrease in aspect ratio) previously observed in the HSPB8_K141N mutant motor neurons **(Figure 2C).**

Additionally, the K141 residue seems to play an important role in the interaction of HSPB8 with BAG3 since its mutation alters the binding affinity of HSPB8 towards BAG3 (Carra et al. 2010; Shemetov and Gusev 2011; Echaniz-Laguna et al. 2017). A decrease in BAG3 levels was observed in iPSC-derived motor neurons for the HSPB8_K141N mutation (Van Lent et al. 2021) and in sciatic nerve of 12-month-old symptomatic Hspb8[K141N/K141N] mice (Bouhy et al. 2018), likely reflecting a dominant negative effect of mutant HSPB8 and/or a compromised protein quality control. Knockdown of HSPB8 with mi3UTR was able to restore BAG3 protein levels to control (Figure 2D). Note that miSCR also slightly increased BAG3, which could be related to the lentiviral transduction and stable overexpression of the shRNA constructs, thereby introducing a stress response that might in turn induce BAG3 expression (Sturner and Behl 2017). Using higher viral titers in an attempt to reduce HSPB8 expression even further (to more than 50%) led to a collapse of the neurite network, irrespective of the introduced shRNA sequence **(Figure 6).** In summary, silencing of HSPB8 in patient iPSC-derived motor neurons with a heterozygous HSPB8_K141N mutation rescued aberrant mitochondrial morphology and restored BAG3 levels.

Example 3: Recombinant AAV9.miRNA HSPB8 delivery holds potential for improving motor performance *in vivo*

For long-term expression *in vivo,* several studies have demonstrated that target sequence expression via natural microRNA (miRNA) scaffolds is equally or even more potent and less toxic in driving target knock-down than RNA polymerase-III expressed shRNAs (Boudreau, Monteys, and Davidson 2008; McBride et al. 2008; Shan et al. 2008; Silva et al. 2005). Moreover, miRNA expression can be driven by RNA polymerase-II promoters, which allows; i) to link their expression to marker genes, and ii) the use of cell type specific promoters (Stegmeier et al. 2005; Merlin and Follenzi 2019). To this end, the cDNAs of the respective shRNAs were cloned into a miRNA vector backbone and remained efficient in suppressing *HSPB8/Hspb8* expression across human and mouse cell lines **(Figure 7).**

The direct use of lentiviral vectors in the treatment of patients is limited due to the mutagenic risk through genome integration (Hargrove et al. 2008; Bulcha et al. 2021). Moreover, in a gene therapy approach for a demyelinating form of CMT, higher expression rates were achieved with adeno-associated virus (AAV)-mediated delivery compared to lentiviral vectors (Kagiava et al. 2021; Kagiava et al. 2018; Kagiava et al. 2019; Kagiava et al. 2016). During the last decades, AAV vectors have been extensively used for gene delivery, mainly to the central nervous system (CNS), but also to the peripheral nervous system (PNS)

(reviewed in Hudry and Vandenberghe (2019)). We therefore cloned our miRNA scaffolds into an AAV transfer plasmid, downstream of the neuron-specific human synapsin promoter (CMVenh-hSyn) and enhanced green fluorescent protein (GFP). To verify the functionality of our construct and its efficient delivery towards targeted cells, we sought to look at the GFP signal in different tissues harvested from screen pilot mutant mice (Figure 3A). Recombinant AAV2/9 harbouring miSCR were delivered via a single lumbar (between L5 and L6) intrathecal injection into 3-month-old mutant animals. Results showed a positive expression of GFP in both the spinal cord and sciatic nerve.

Behavioural testing was performed on treated Hspb8$^{K141N/K141N}$ mice next to the untreated healthy control animals. To assess motor performance and electrophysiological disease progression, animals were subjected to accelerating rotarod testing and compound muscle action potential (CMAP) measurements from 3 months (prior to the treatment) until 12 months of age (Figure 3B). The first round of behavioural testing showed no differences between WT and Hspb8$^{K141N/K141N}$ mice, indicating that at this age the mutant mice were asymptomatic (Figure 3C and D). Throughout the experimental time, the average time spend on the rotarod revealed that mi3UTR and mi559 treated Hspb8$^{K141N/K141N}$ mice performed as good as the untreated healthy controls (Figure 3C), while mice treated with miSCR tend to slightly decrease in performance over aging. No additional stress factor was included in this behavioural experiment, and the shy difference observed in the miSCR treated mice did not reflect our previous reported data demonstrating that untreated Hspb8$^{K141N/K141N}$ performed poorly on the rotarod overtime when compared to wild type mice (Bouhy et al. 2018). We could speculate that the current rotarod data depicts a possible exercise adaptation of the animals to the rotarod device. On the other hand, no improvement in CMAP could be observed after treatment in any mutant mice compared to healthy untreated animals. However, within 6 months old treated mutant mice, a slight statistically non-significant improvement of the mean CMAP amplitudes could be detected with the mi3UTR followed by the mi559 compared to miSCR, indicating a possible slowdown in the disease progression in the first 3 months post-treatment (Figure 3D). Although the behavioural and electrophysiological testing were performed every 3 months with the same groups of animals (treated versus non-treated), we could not appreciate any clear benefit of the mi3UTR and mi599 AAV9 injected constructs. To allow the identification of a more detectable and unbiased therapeutic effect of the miRNA-AAV9 constructs, we proceeded with *in vivo* magnetic resonance imaging (MRI) and a neuromuscular histopathological study as end-point of the treated animals.

Example 4: MRI analysis monitors neuromuscular features of untreated healthy control mice and RNA interference treated Hspb8 knock-in mice.

To further assess the therapeutic effect on muscle and nerve pathology we performed *in vivo* MRI at the age of 12 months. Different MRI scans were acquired to evaluate different aspects of the pathology and potential treatment effect. First, we evaluated the severity of the sciatic nerve damage, and whether the knock-down treatments with the RNA interference AAV2/9 constructs would improve the nerve integrity by acquiring diffusion weighted images (DWI) and $T_2$ maps (Figure 4A-B, E). The sciatic nerve of miSCR treated Hspb8$^{K141N/K141N}$ mice have decreased fractional anisotropy (FA) values and increased $T_2$ values compared to WT control mice, indicative of nerve damage. Note that more in depth analysis of the other diffusion parameters could not pin-point these FA changes to specific radial or axial diffusivity changes (Figure 9). Among the two putative therapeutic shRNAs tested, only mi3UTR was able to increase the FA value and more significantly reduce the $T_2$ value, suggesting its potential in preventing sciatic nerve damage.

Secondly, multi-slice anatomical scans were acquired to determine the volume of the calf muscle, since nerve damage ultimately leads to muscle atrophy. Results showed that muscle volume was reduced from $241 \pm 19$ mm$^3$ (Mean $\pm$SD) in healthy WT control mice to $168 \pm 8$ mm$^3$ in Hspb8 $^{K141N/K141N}$ treated mice (Figure 4C, F), indicating that none of the treatments were able to depict a therapeutic benefit in rescuing muscle atrophy. Additionally, $T_2$ maps of the calf muscles revealed an increased $T_2$ value in all treated Hspb8 mutant mice compared to healthy WT control animals, indicative of lesions in the muscle that could not be reverted (Figure 4D). The MRI data, showing the muscle atrophy, corresponds with decreasing CMAP amplitudes over time in treated and non-treated Hspb8 knock-in animals (Figure 3D).

After the MRI analysis, all used animals were euthanized and tissues were dissected and collected as end-point experiment. Western blotting was performed on three different tissues (spinal cord, sciatic nerve and gastrocnemius muscle) to verify the intrathecal AAV2/9 injection through long-term expression of the GFP-miRNA constructs. The GFP signal confirmed successful injections of the miSCR, mi3UTR and mi559 AAV2/9 constructs in 3 Hspb8 knock-in animals involved in the MRI study (Figure 8). Results showed that the constructs were predominantly located in the spinal cord in comparison with the sciatic nerve. None of the treated mice showed GFP signal in the muscle, confirming the specificity of the AAV2/9 vector to neuronal cells. Furthermore, in the lumbar part of the spinal cord the location of GFP-expressing cells was evaluated on cross-sections with microscopy after immunohistological staining with a fluorescently labelled anti-GFP nanobody. GFP-positive cells could be detected after injection with all constructs, and were mostly located at the outer borders of the spinal cord, with reducing Hspb8 expression to almost 50% with the mi3UTR shRNA containing vector

**(Figure 8).**

Example 5: Histopathology study of the mi3UTR shRNA treatment in the Hspb8 knock-in mouse model.

We selected representative TEM images to illustrate the sciatic nerve pathology of treated mice. We observed pronounced osmophilic intra-axonal aggregates of amorphous and granular material as well as vesicular structures resembling smooth ER and abnormal, swollen mitochondria with signs of mitophagy **(Figure 5A).** Several myelin ovoids and myelinophages containing myelin debris were indicative of a complete breakdown of myelinated nerve fibers **(Figure 5B).** There were few clusters of regenerating axons **(Figure 5C).** Increased numbers of nerve fibers with excessive myelin foldings were occasionally found and are presumed to be secondary signs of axonal degeneration **(Figure 5D).** There were signs of a moderate loss also of non-myelinated axons **(Figure 5E).** Occasionally, thinly myelinated axons with redundant basal lamina loops indicative of de- and remyelination could be seen in all of the treated animals **(Figure 5F).** Altogether, these histopathological features were very similar to those previously reported in non-treated homozygous Hspb8$^{K141N/K141N}$ knock-in mice (Bouhy et al. 2018). However, when we quantified the neuromuscular features we found that the percentage of atrophic muscle fibers could be reduced almost by half with the mi3UTR compared to miSCR treated animals **(Figure 5G).** Yet, this amelioration was not sufficient to revert to a normal phenotype. On the other hand, because AAV and shRNA are both known to potentially induce an innate immune response we investigated whether the RNA therapy would trigger nerve fibre breakdown by counting myelin ovoids and myelinophages in nerve sections of treated and untreated healthy control animals. Results showed that macrophages were only detected in treated animals indicating an activation of the immune system upon intrathecal injections of the miRNA constructs in the spinal cord **(Figure 5H).** Also, the number of focally folded myelin per $\mu$m$^2$ was only and partially decreased in the sciatic nerves treated with the mi3UTR compared to miSCR treated animals (Figure 5I). In addition, a decrease in number of Hspb8 axonal aggregates per $\mu$m$^2$ could also be appreciated with the mi3UTR and mi559 constructs, compared to miSCR treated animals **(Figure 5J).** These quantitative pathology data demonstrate a partial beneficial effect of the mi3UTR AAV2/9 when compared to miSCR AAV2/9 treated Hspb8 knock-in animals.

Although the quantification of muscle and nerve pathologies somewhat aligned with the behavioral and MRI studies, i.e. decreased numbers of myelin foldings and atrophic muscle fibers in mi3UTR-treated animals compared to the miSCR-treated ones, a statistically significant correlation with the shRNA AAV treatment could not be established due to the low number of animals in each group at this end-point experiment.

**References**

Boudreau, R. L., A. M. Monteys, and B. L. Davidson. 2008. 'Minimizing variables among hairpin-based RNAi vectors reveals the potency of shRNAs', RNA, 14: 1834-44.

Bouhy, D., M. Juneja, I. Katona, A. Holmgren, B. Asselbergh, V. De Winter, T. Hochepied, S. Goossens, J.J. Haigh, C. Libert, C. Ceuterick-de Groote, J. Irobi, J. Weis, and V. Timmerman. 2018. 'A knock-in/knock-out mouse model of HSPB8-associated distal hereditary motor neuropathy and myopathy reveals toxic gain-of-function of mutant Hspb8', Acta Neuropathol, 135: 131-48.

Bulcha, Jote T., Yi Wang, Hong Ma, Phillip W. L. Tai, and Guangping Gao. 2021. 'Viral vector platforms within the gene therapy landscape', Signal Transduction and Targeted Therapy, 6: 53.

Carra, S., A. Boncoraglio, B. Kanon, J.F. Brunsting, M. Minoia, A. Rana, M.J. Vos, K. Seidel, O.C. Sibon, and H.H. Kampinga. 2010. 'Identification of the Drosophila ortholog of HSPB8: implication of HSPB8 loss of function in protein folding diseases', J Biol.Chem., 285: 37811-22.

Charles, James P., Ornella Cappellari, Andrew J. Spence, John R. Hutchinson, and Dominic J. Wells. 2016. 'Musculoskeletal Geometry, Muscle Architecture and Functional Specialisations of the Mouse Hindlimb', PLOS ONE, 11: e0147669.

Echaniz-Laguna, A., T. Geuens, P. Petiot, Y. Pereon, E. Adriaenssens, M. Haidar, S. Capponi, T. Maisonobe, E. Fournier, O. Dubourg, B. Degos, F. Salachas, T. Lenglet, B. Eymard, E. Delmont, J. Pouget, R. Juntas Morales, C. Goizet, P. Latour, V. Timmerman, and T. Stojkovic. 2017. 'Axonal Neuropathies due to Mutations in Small Heat Shock Proteins: Clinical, Genetic, and Functional Insights into Novel Mutations', Hum Mutat, 38: 556-68.

Ghaoui, R., J. Palmio, J. Brewer, M. Lek, M. Needham, A. Evila, P. Hackman, P. H. Jonson, S. Penttila, A. Vihola, S. Huovinen, M. Lindfors, R. L. Davis, L. Waddell, S. Kaur, C. Yiannikas, K. North, N. Clarke, D. G. MacArthur, C. M. Sue, and B. Udd. 2016. 'Mutations in HSPB8 causing a new phenotype of distal myopathy and motor neuropathy', Neurology, 86: 391-8.

Groh, J., J. Weis, H. Zieger, E. R. Stanley, H. Heuer, and R. Martini. 2012. 'Colony-stimulating factor-1 mediates macrophage-related neural damage in a model for Charcot-Marie-Tooth disease type 1X', Brain, 135: 88-104.

Guo, W., M. Naujock, L. Fumagalli, T. Vandoorne, P. Baatsen, R. Boon, L. Ordovas, A. Patel, M. Welters, T. Vanwelden, N. Geens, T. Tricot, V. Benoy, J. Steyaert, C. Lefebvre-Omar, W. Boesmans, M. Jarpe, J. Sterneckert, F. Wegner, S. Petri, D. Bohl, P. Vanden Berghe, W. Robberecht, P. Van Damme, C. Verfaillie, and L. Van Den Bosch. 2017. 'HDAC6 inhibition reverses axonal transport defects in motor neurons derived from FUS-ALS patients', Nat Commun, 8: 861.

Hargrove, Phillip W., Steven Kepes, Hideki Hanawa, John C. Obenauer, Deiqing Pei, Cheng Cheng, John T. Gray, Geoffrey Neale, and Derek A. Persons. 2008. 'Globin Lentiviral Vector Insertions Can Perturb the Expression of Endogenous Genes in β-thalassemic Hematopoietic Cells', Molecular Therapy, 16: 525-33.

Hellemans, Jan, Geert Mortier, Anne De Paepe, Frank Speleman, and Jo Vandesompele. 2007. 'qBase relative quantification framework and software for management and automated analysis of real-time quantitative PCR data', Genome Biology, 8: R19.

Huang, Lei, Jin-Na Min, Shane Masters, Nahid F. Mivechi, and Demetrius Moskophidis. 2007. 'Insights into function and regulation of small heat shock protein 25 (HSPB1) in a mouse model with targeted gene disruption', genesis, 45: 487-501.

Hudry, Eloise, and Luk H. Vandenberghe. 2019. 'Therapeutic AAV Gene Transfer to the Nervous System: A Clinical Reality', Neuron, 101: 839-62.

Huylebroeck, Danny, Geert Maertens, Martine Verhoeyen, Claude Lopez, Alex Raeymakers, Willy Min Jou, and Walter Fiers. 1988. 'High-level transient expression of influenza virus proteins from a series of SV40 late and early replacement vectors', Gene, 66: 163-81.

Hylden, Janice L. K., and George L. Wilcox. 1980. 'Intrathecal morphine in mice: A new technique', European Journal of Pharmacology, 67: 313-16.

Kagiava, A., C. Karaiskos, J. Richter, C. Tryfonos, G. Lapathitis, I. Sargiannidou, C. Christodoulou, and K. A. Kleopa. 2018. 'Intrathecal gene therapy in mouse models expressing CMT1X mutations', Human Molecular Genetics, 27: 1460-73.

Kagiava, A., J. Richter, C. Tryfonos, C. Karaiskos, A. J. Heslegrave, I. Sargiannidou, A. M. Rossor, H. Zetterberg, M. M. Reilly, C. Christodoulou, and K. A. Kleopa. 2019. 'Gene replacement therapy after neuropathy onset provides therapeutic benefit in a model of CMT1X', Human Molecular Genetics, 28: 3528-42.

Kagiava, Alexia, Christos Karaiskos, Jan Richter, Christina Tryfonos, Matthew J. Jennings, Amanda J. Heslegrave, Irene Sargiannidou, Marina Stavrou, Henrik Zetterberg, Mary M. Reilly, Christina Christodoulou, Rita Horvath, and Kleopas A. Kleopa. 2021. 'AAV9-mediated Schwann cell-targeted gene therapy rescues a model of demyelinating neuropathy', Gene Therapy, 28: 659-75.

Kagiava, Alexia, Irene Sargiannidou, George Theophilidis, Christos Karaiskos, Jan Richter, Stavros Bashiardes, Natasa Schiza, Marianna Nearchou, Christina Christodoulou, Steven S. Scherer, and Kleopas A. Kleopa. 2016. 'Intrathecal gene therapy rescues a model of demyelinating peripheral neuropathy', Proceedings of the National Academy of Sciences, 113: E2421-E29.

Kammoun, M., B. Picard, T. Astruc, M. Gagaoua, D. Aubert, M. Bonnet, V. Blanquet, and I. Cassar-Malek. 2016. 'The Invalidation of HspB1 Gene in Mouse Alters the Ultrastructural Phenotype of Muscles', Plos One, 11: e0158644.

McBride, Jodi L., Ryan L. Boudreau, Scott Q. Harper, Patrick D. Staber, Alex Mas Monteys, Inâs Martins, Brian L. Gilmore, Haim Burstein, Richard W. Peluso, Barry Polisky, Barrie J. Carter, and Beverly L. Davidson. 2008. 'Artificial miRNAs mitigate shRNA-mediated toxicity in the brain: Implications for the therapeutic development of RNAi', Proceedings of the National Academy of Sciences, 105: 5868-73.

Merlin, Simone, and Antonia Follenzi. 2019. 'Transcriptional Targeting and MicroRNA Regulation of Lentiviral Vectors', Molecular Therapy - Methods & Clinical Development, 12: 223-32.

Osório, Luísa, Rik Gijsbers, Marusela Oliveras-Salvá, Annelies Michiels, Zeger Debyser, Chris Van den Haute, and Veerle Baekelandt. 2014. 'Viral vectors expressing a single microRNA-based short-hairpin RNA result in potent gene silencing in vitro and in vivo', Journal of Biotechnology, 169: 71-81.

Pollari, Eveliina, Robert Prior, Wim Robberecht, Philip Van Damme, and Ludo Van Den Bosch. 2018. 'In Vivo Electrophysiological Measurement of Compound Muscle Action Potential from the Forelimbs in Mouse Models of Motor Neuron Degeneration', JoVE: e57741.

Rauch, J. N., E. Tse, R. Freilich, S. A. Mok, L. N. Makley, D. R. Southworth, and J. E. Gestwicki. 2017. 'BAG3 Is a Modular, Scaffolding Protein that physically Links Heat Shock Protein 70 (Hsp70) to the Small Heat Shock Proteins', J Mol Biol, 429: 128-41.

Schindelin, J., I. Arganda-Carreras, E. Frise, V. Kaynig, M. Longair, T. Pietzsch, S. Preibisch, C. Rueden, S. Saalfeld, B. Schmid, J. Y. Tinevez, D. J. White, V. Hartenstein, K. Eliceiri, P. Tomancak, and A. Cardona. 2012. 'Fiji: an open-source platform for biological-image analysis', Nat Methods, 9: 676-82.

Schneider, C.A., W.S. Rasband, and K.W. Eliceiri. 2012. 'NIH Image to ImageJ: 25 years of image analysis', Nat Methods, 9: 671-75.

Shan, Ge, Yujing Li, Junliang Zhang, Wendi Li, Keith E. Szulwach, Ranhui Duan, Mohammad A. Faghihi, Ahmad M.

Khalil, Lianghua Lu, Zain Paroo, Anthony W. S. Chan, Zhangjie Shi, Qinghua Liu, Claes Wahlestedt, Chuan He, and Peng Jin. 2008. 'A small molecule enhances RNA interference and promotes microRNA processing', Nature Biotechnology, 26: 933-40.

Shemetov, A. A., and N. B. Gusev. 2011. 'Biochemical characterization of small heat shock protein HspB8 (Hsp22)-Bag3 interaction', Arch Biochem Biophys, 513: 1-9.

Silva, Jose M., Mamie Z. Li, Ken Chang, Wei Ge, Michael C. Golding, Richard J. Rickies, Despina Siolas, Guang Hu, Patrick J. Paddison, Michael R. Schlabach, Nihar Sheth, Jeff Bradshaw, Julia Burchard, Amit Kulkarni, Guy Cavet, Ravi Sachidanandam, W. Richard McCombie, Michele A. Cleary, Stephen J. Elledge, and Gregory J. Hannon. 2005. 'Second-generation shRNA libraries covering the mouse and human genomes', Nature Genetics, 37: 1281-88.

Stavrou, M., I. Sargiannidou, T. Christofi, and K. A. Kleopa. 2021. 'Genetic mechanisms of peripheral nerve disease', Neurosci Lett, 742: 135357.

Stegmeier, Frank, Guang Hu, Richard J. Rickles, Gregory J. Hannon, and Stephen J. Elledge. 2005. 'A lentiviral microRNA-based system for single-copy polymerase II-regulated RNA interference in mammalian cells', Proceedings of the National Academy of Sciences, 102: 13212-17.

Sturner, E., and C. Behl. 2017. 'The Role of the Multifunctional BAG3 Protein in Cellular Protein Quality Control and in Disease', Front Mol Neurosci, 10: 177.

Sun, Daqian, Margherita Melegari, Sunandini Sridhar, Charles E. Rogler, and Liang Zhu. 2006. 'Multi-miRNA hairpin method that improves gene knockdown efficiency and provides linked multi-gene knockdown', BioTechniques, 41: 59-63.

Tedesco, B., L. Vendredy, V. Timmerman, and A. Poletti. 2023. 'The chaperone-assisted selective autophagy complex dynamics and dysfunctions', Autophagy, 19: 1619-41.

Tournier, J. D., R. Smith, D. Raffelt, R. Tabbara, T. Dhollander, M. Pietsch, D. Christiaens, B. Jeurissen, C. H. Yeh, and A. Connelly. 2019. 'MRtrix3: A fast, flexible and open software framework for medical image processing and visualisation', Neuroimage, 202: 116137.

Van der Perren, A., J. Toelen, M. Carlon, C. Van den Haute, F. Coun, B. Heeman, V. Reumers, L. H. Vandenberghe, J. M. Wilson, Z. Debyser, and V. Baekelandt. 2011. 'Efficient and stable transduction of dopaminergic neurons in rat substantia nigra by rAAV 2/1, 2/2, 2/5, 2/6.2, 2/7, 2/8 and 2/9', Gene Therapy, 18: 517-27.

Van Lent, J.; Prior, P.; Pérez Siles, G.; Cutrupi, A.N.; Kennerson, M.L.; Vangansewinkel, T.; Wolfs, E.; Mukherjee-Clavin, B.; Judge, L.; Conklin, B.; Tyynismaa, H.; Clark, A.J.; Bennett, D.; Van Den Bosch, L.; Saporta, M.; Timmerman, V. 2024. "Advances and challenges in modeling inherited peripheral neuropathies using iPSCs." In Experimental Molecular Medicine.

Xu, Jianming. 2005. 'Preparation, Culture, and Immortalization of Mouse Embryonic Fibroblasts', Current Protocols in Molecular Biology, 70: 28.1.1-28.1.8.

Yang, X. D., Z. D. Cen, H. P. Cheng, K. Shi, J. Bai, F. Xie, H. W. Wu, B. B. Li, and W. Luo. 2017. 'L-3-n-Butylphthalide Protects HSPB8 K141N Mutation-Induced Oxidative Stress by Modulating the Mitochondrial Apoptotic and Nrf2 Pathways', Front Neurosci, 11: 402.

## Claims

1. An RNA interference (RNAi) oligonucleotide for inhibiting expression of *HSPB8* in a cell, wherein the oligonucleotide comprises a guide strand sequence that is complementary to at least 15, 16, 17, 18, or 19 contiguous bases in the 3' UTR region of the human *HSPB8* transcript (SEQ ID NO: 1).

2. An oligonucleotide according to claim 1, wherein the guide strand sequence is complementary to at least 15, 16, 17, 18, or 19 contiguous bases in the base sequence 5'-AGGAACCAAGCAAAGGCCAG-'3 (SEQ ID NO: 2).

3. An oligonucleotide according to claim 1 or 2, wherein the oligonucleotide further comprises a passenger strand sequence that shares a region of complementarity of at least 15, 16, 17, 18, or 19 bases with the guide strand.

4. An oligonucleotide according to any one of the preceding claims, wherein the oligonucleotide is a single stranded siRNA or a double stranded siRNA, preferably wherein the oligonucleotide comprises or consists of the base sequence of SEQ ID NO: 2 or 3.

5. An oligonucleotide according to claim 4, wherein the length of the oligonucleotide is from 19 to 30 nucleotides.

6. An oligonucleotide according to any one of claims 1-3, wherein the oligonucleotide is a short hairpin RNA (shRNA) or an artificial microRNA (amiRNA), preferably wherein the oligonucleotide comprises or consists of the base sequence of SEQ ID NO: 5.

7. An oligonucleotide according to claim 6, wherein the length of the oligonucleotide is from 40 to 80, preferably from 50 to 70 nucleotides.

8. An oligonucleotide according to any one of the preceding claims, wherein the oligonucleotide comprises one or more modified or artificial internucleoside linkages, one or more modified or artificial sugars, and/or one or more modified or artificial bases.

9. An expression vector encoding an oligonucleotide as described in any one of claims 1-7.

10. An expression vector according to claim 9, wherein the expression vector is a viral vector, preferably an adeno-associated viral (AAV) vector.

11. A pharmaceutical composition comprising an oligonucleotide as described in any one of claims 1-8 or an expression vector according to claim 9 or 10.

12. An oligonucleotide according to any one of claims 1-8, an expression vector according to claim 9 or 10, or a pharmaceutical composition according to claim 11, for use in medicine.

13. An oligonucleotide according to any one of claims 1-8, an expression vector according to claim 9 or 10, or a pharmaceutical composition according to claim 11, for use in treating or preventing a disease selected from the group consisting of a motor neuropathy, an axonal CMT neuropathy, and a distal myopathy, optionally wherein the disease is associated with a mutation in the *HSPB8* gene.

14. An oligonucleotide, expression vector, or pharmaceutical composition for use according to claim 13, wherein the motor neuropathy is distal hereditary motor neuropathy (dHMN), the axonal CMT neuropathy is Charcot-Marie-Tooth disease subtype 2L (CMT2L), and the distal myopathy is myofibrillar myopathy (MFM).

15. A method for inhibiting expression of *HSPB8* in a cell, said method comprising contacting the cell with an oligonucleotide as described in any one of claims 1-8 or an expression vector according to claim 9 or 10, preferably wherein the method is an *in vitro* or an *ex vivo* method.

**FIG 1**

**FIG 2**

FIG 3

FIG 3 (continued)

**FIG 4**

E

WT control

Hspb8 + miSCR

Hspb8 + mi559

Hspb8 +mi3UTR

F

WT control

Hspb8

FIG 4 (continued)

FIG 5

FIG 5 (continued)

FIG 6

**B**

Control

HSPB8_K141N

HSPB8_K141N
+ miSCR (low)

HSPB8_K141N + miSCR (high)

HSPB8_K141N
+ mi3UTR (low)

HSPB8_K141N + mi3UTR (high)

FIG 6 (continued)

**FIG 7**

**FIG 8**

**FIG 8 (continued)**

FIG 9

Europäisches Patentamt

European Patent Office

Office européen des brevets

## EUROPEAN SEARCH REPORT

**Application Number**

EP 24 18 5813

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | FUCHS MARGIT ET AL: "A Role for the Chaperone Complex BAG3-HSPB8 in Actin Dynamics, Spindle Orientation and Proper Chromosome Segregation during Mitosis", PLOS GENETICS, vol. 11, no. 10, 23 October 2015 (2015-10-23), page e1005582, XP093235916, US ISSN: 1553-7404, DOI: 10.1371/journal.pgen.1005582 Retrieved from the Internet: URL:https://journals.plos.org/plosgenetics /article/file?id=10.1371/journal.pgen.1005 582&type=printable> | 1-11 | INV. C12N15/113 A61K31/7088 |
| Y | * page 22; figure 5 * ----- | 12-15 | |
| Y | Geneeskundige Stichting Koningin Elisabeth (g.S.K.E.): "Verslag - Rapport - Bericht - Report - 2019 Geneeskundige Stichting Koningin Elisabeth (G.S.K.E.)", , 19 April 2022 (2022-04-19), pages 1-228, XP093236189, Retrieved from the Internet: URL:https://web.archive.org/web/2022041918 0921if_/https://www.fmre-gske.be/pdf/jaarv erslag_2019.pdf * pages 115, 116 * ----- | 12-15 | |
| | -/-- | | |

| | | | TECHNICAL FIELDS SEARCHED (IPC) |
|---|---|---|---|
| | | | C12N A61K |

The present search report has been drawn up for all claims

| Place of search | Date of completion of the search | Examiner |
|---|---|---|
| The Hague | 10 January 2025 | Harvey, Maia |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 1 of 3

Europäisches
Patentamt

European
Patent Office

Office européen
des brevets

## EUROPEAN SEARCH REPORT

**Application Number**

EP 24 18 5813

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | SHERER: "2018 Peripheral Nerve Society Annual Meeting 21-25 July, 2018 Baltimore, Maryland", JOURNAL OF THE PERIPHERAL NERVOUS SYSTEM, vol. 23, no. 4, 1 December 2018 (2018-12-01), pages 249-405, XP055676584, US ISSN: 1085-9489, DOI: 10.1111/jns.12290 * Poster no. 113 * | 1-15 | |
| A | YAO SHAOMIAN ET AL: "Expression of microRNAs targeting heat shock protein B8 during in vitro expansion of dental pulp stem cells in regulating osteogenic differentiation", ARCHIVES OF ORAL BIOLOGY, PERGAMON PRESS, OXFORD, GB, vol. 107, 22 July 2019 (2019-07-22), XP085856523, ISSN: 0003-9969, DOI: 10.1016/J.ARCHORALBIO.2019.104485 [retrieved on 2019-07-22] * figures 1, 2 * | 1-15 | |
| A | SENGAR GYANENDRA SINGH ET AL: "Differential expression of microRNAs associated with thermal stress in Frieswal (Bos taurusxBos indicus) crossbred dairy cattle", CELL STRESS AND CHAPERONES, ALLEN PRESS ONLINE PUBLISHING, EDINBURGH, GB, vol. 23, no. 1, 3 August 2017 (2017-08-03) , pages 155-170, XP036386654, ISSN: 1355-8145, DOI: 10.1007/S12192-017-0833-6 [retrieved on 2017-08-03] * figures 6, 8 * | 1-15 | TECHNICAL FIELDS SEARCHED (IPC) |

-/--

The present search report has been drawn up for all claims

| Place of search | Date of completion of the search | Examiner |
|---|---|---|
| The Hague | 10 January 2025 | Harvey, Maia |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

**EUROPEAN SEARCH REPORT**

Application Number

EP 24 18 5813

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | CN 107 022 545 A (JIANG BIMEI) 8 August 2017 (2017-08-08) * paragraphs [0010], [0025], [0053] – [0055] * ----- | 1-15 | |
| | | | TECHNICAL FIELDS SEARCHED (IPC) |

The present search report has been drawn up for all claims

| Place of search | Date of completion of the search | Examiner |
|---|---|---|
| The Hague | 10 January 2025 | Harvey, Maia |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

.........................................................................................

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 18 5813

This annex lists the patent family members relating to the patent documents cited in the above-mentioned European search report.
The members are as contained in the European Patent Office EDP file on
The European Patent Office is in no way liable for these particulars which are merely given for the purpose of information.

10-01-2025

| Patent document cited in search report | Publication date | Patent family member(s) | Publication date |
|---|---|---|---|
| CN 107022545 A | 08-08-2017 | NONE | |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6683173 B

**Non-patent literature cited in the description**

- Synthesis of Therapeutic Oligonucleotides. Springer, 2018
- Design and Delivery of siRNA Therapeutics.. Methods in Molecular Biology. 2021
- **LIMA, W.F. et al.** *Cell*, 2012, vol. 150, 883-894
- **YU, D.** *Cell*, 2012, vol. 150, 895-908
- **KOTOWSKA-ZIMMER et al.** Artificial miRNAs as therapeutic tools: Challenges and opportunities. *Wiley Interdiscip Rev RNA.*, July 2021, vol. 12 (4), e1640
- **BISCANS et al.** *Bioorg. Med. Chem.*, 2015, vol. 23, 5360
- **YAMADA et al.** *Org. Biomol. Chem.*, 2014, vol. 12, 6457
- **ARAI K. et al.** *Bioorg. Med. Chem.*, 2011, vol. 21, 6285
- **ØSTERGAARD et al.** *ACS Chem. Biol.*, 2014, vol. 22, 6227
- **AUSUBEL et al.** Current Protocols in Molecular Biology. John Wiley & Sons Inc, 2003
- **SAMBROOK** ; **GREEN**. Molecular Cloning: A Laboratory Manual. Cold Spring Harbor Laboratory Press, 2012
- Remington: The Science and Practice of Pharmacy. Elsevier, 2020
- **AUSUBEL et al.** Current Protocols in Molecular Biology. John Wiley & Sons, Inc, 2003
- **SAMBROOK** ; **GREEN**. Molecular Cloning. A Laboratory Manual. Cold Spring Harbor Laboratory Press, 2012
- Bioinformatics and the Cell: Modern Computational Approaches in Genomics. **XIA X**. Proteomics and transcriptomics. Springer International Publishing, 2018
- **MOUNT D.** Bioinformatics: Sequence and Genome Analysis. Cold Spring Harbor Laboratory Press, 2004
- **HENIKOFF** ; **HENIKOFF**. *PNAS*, 1992, vol. 89, 915-919
- **ALTSCHUL et al.** *J. Mol. Biol.*, 1990, vol. 215, 403-10
- **ALTSCHUL et al.** *Nucleic Acids Res.*, 1997, vol. 25 (17), 3389-3402

- **BOUDREAU, R. L** ; **A. M. MONTEYS** ; **B. L. DAVIDSON**. Minimizing variables among hairpin-based RNAi vectors reveals the potency of shRNAs. *RNA*, 2008, vol. 14, 1834-44
- **BOUHY, D** ; **M. JUNEJA** ; **I. KATONA** ; **A. A. HOLMGREN** ; **B. ASSELBERGH** ; **V. DE WINTER** ; **T. HOCHEPIED** ; **S. GOOSSENS** ; **J.J. HAIGH** ; **C. LIBERT**. A knock-in/knock-out mouse model of HSPB8-associated distal hereditary motor neuropathy and myopathy reveals toxic gain-of-function of mutant Hspb8. *Acta Neuropathol*, 2018, vol. 135, 131-48
- **BULCHA, JOTE T.** ; **YI WANG** ; **HONG MA** ; **PHILLIP W. L. TAI** ; **GUANGPING GAO.** Viral vector platforms within the gene therapy landscape. *Signal Transduction and Targeted Therapy*, 2021, vol. 6, 53
- **CARRA, S** ; **A. BONCORAGLIO** ; **B. KANON** ; **J.F. BRUNSTING** ; **M. MINOIA** ; **A. RANA** ; **M.J. VOS** ; **K. SEIDEL** ; **O.C. SIBON** ; **H.H. KAMPINGA**. Identification of the Drosophila ortholog of HSPB8: implication of HSPB8 loss of function in protein folding diseases. *J Biol.Chem.*, 2010, vol. 285, 37811-22
- **CHARLES, JAMES P** ; **ORNELLA CAPPELLARI** ; **ANDREW J. SPENCE** ; **JOHN R. HUTCHINSON** ; **DOMINIC J. WELLS.** Musculoskeletal Geometry, Muscle Architecture and Functional Specialisations of the Mouse Hindlimb. *PLOS ONE*, 2016, vol. 11, e0147669
- **ECHANIZ-LAGUNA, A.** ; **T. GEUENS** ; **P. PETIOT** ; **Y. PEREON** ; **E. ADRIAENSSENS** ; **M. HAIDAR** ; **S. CAPPONI** ; **T. MAISONOBE** ; **E. FOURNIER** ; **O. DUBOURG**. Axonal Neuropathies due to Mutations in Small Heat Shock Proteins: Clinical, Genetic, and Functional Insights into Novel Mutations. *Hum Mutat*, 2017, vol. 38, 556-68
- **GHAOUI, R.** ; **J. PALMIO** ; **J. BREWER** ; **M. LEK** ; **M. NEEDHAM** ; **A. EVILA** ; **P. HACKMAN** ; **P. H. JONSON** ; **S. PENTTILA** ; **A. VIHOLA**. Mutations in HSPB8 causing a new phenotype of distal myopathy and motor neuropathy. *Neurology*, 2016, vol. 86, 391-8

- **GROH, J** ; **J. WEIS** ; **H. ZIEGER** ; **E. R. STANLEY** ; **H. HEUER** ; **R. MARTINI.** Colony-stimulating factor-1 mediates macrophage-related neural damage in a model for Charcot-Marie-Tooth disease type 1X. *Brain*, 2012, vol. 135, 88-104
- **GUO, W.** ; **M. NAUJOCK** ; **L. FUMAGALLI** ; **T. VANDOORNE** ; **P. BAATSEN** ; **R. BOON** ; **L. ORDOVAS** ; **A. PATEL** ; **M. WELTERS** ; **T. VANWELDEN.** HDAC6 inhibition reverses axonal transport defects in motor neurons derived from FUS-ALS patients. *Nat Commun*, 2017, vol. 8, 861
- **HARGROVE, PHILLIP W.** ; **STEVEN KEPES** ; **HIDEKI HANAWA** ; **JOHN C. OBENAUER** ; **DEIQING PEI** ; **CHENG CHENG** ; **JOHN T. GRAY** ; **GEOFFREY NEALE** ; **DEREK A. PERSONS.** Globin Lentiviral Vector Insertions Can Perturb the Expression of Endogenous Genes in β-thalassemic Hematopoietic Cells. *Molecular Therapy*, 2008, vol. 16, 525-33
- **HELLEMANS, JAN** ; **GEERT MORTIER** ; **ANNE DE PAEPE** ; **FRANK SPELEMAN** ; **JO VANDESOMPELE.** qBase relative quantification framework and software for management and automated analysis of real-time quantitative PCR data. *Genome Biology*, 2007, vol. 8, R19
- **HUANG, LEI** ; **JIN-NA MIN** ; **SHANE MASTERS** ; **NAHID F. MIVECHI** ; **DEMETRIUS MOSKOPHIDIS.** Insights into function and regulation of small heat shock protein 25 (HSPB1) in a mouse model with targeted gene disruption. *genesis*, 2007, vol. 45, 487-501
- **HUDRY** ; **ELOISE** ; **LUK H. VANDENBERGHE.** Therapeutic AAV Gene Transfer to the Nervous System: A Clinical Reality. *Neuron*, 2019, vol. 101, 839-62
- **HUYLEBROECK** ; **DANNY** ; **GEERT MAERTENS** ; **MARTINE VERHOEYEN** ; **CLAUDE LOPEZ** ; **ALEX RAEYMAKERS** ; **WILLY MIN JOU** ; **WALTER FIERS.** High-level transient expression of influenza virus proteins from a series of SV40 late and early replacement vectors. *Gene*, 1988, vol. 66, 163-81
- **HYLDEN, JANICE L. K** ; **GEORGE L. WILCOX.** Intrathecal morphine in mice: A new technique. *European Journal of Pharmacology*, 1980, vol. 67, 313-16
- **KAGIAVA, A** ; **C. KARAISKOS** ; **J. RICHTER** ; **C. TRYFONOS** ; **G. LAPATHITIS** ; **I. SARGIANNIDOU** ; **C. CHRISTODOULOU** ; **K. A. KLEOPA.** Intrathecal gene therapy in mouse models expressing CMT1X mutations. *Human Molecular Genetics*, 2018, vol. 27, 1460-73
- **KAGIAVA, A.** ; **J. RICHTER** ; **C. TRYFONOS** ; **C. KARAISKOS** ; **A. J. HESLEGRAVE** ; **I. SARGIANNIDOU** ; **A. M. ROSSOR** ; **H. ZETTERBERG** ; **M. M. REILLY** ; **C. CHRISTODOULOU.** Gene replacement therapy after neuropathy onset provides therapeutic benefit in a model of CMT1X. *Human Molecular Genetics*, 2019, vol. 28, 3528-42
- **KAGIAVA, ALEXIA** ; **CHRISTOS KARAISKOS** ; **JAN RICHTER** ; **CHRISTINA TRYFONOS** ; **MATTHEW J. JENNINGS** ; **AMANDA J. HESLEGRAVE** ; **IRENE SARGIANNIDOU** ; **MARINA STAVROU** ; **HENRIK ZETTERBERG** ; **MARY M. REILLY.** AAV9-mediated Schwann cell-targeted gene therapy rescues a model of demyelinating neuropathy. *Gene Therapy*, 2021, vol. 28, 659-75
- **KAGIAVA, ALEXIA** ; **IRENE SARGIANNIDOU** ; **GEORGE THEOPHILIDIS** ; **CHRISTOS KARAISKOS** ; **JAN RICHTER** ; **STAVROS BASHIARDES** ; **NATASA SCHIZA** ; **MARIANNA NEARCHOU** ; **CHRISTINA CHRISTODOULOU** ; **STEVEN S. SCHERER.** Intrathecal gene therapy rescues a model of demyelinating peripheral neuropathy. *Proceedings of the National Academy of Sciences*, 2016, vol. 113, E2421-E29
- **KAMMOUN, M** ; **B. PICARD** ; **T. ASTRUC** ; **M. GAGAOUA** ; **D. AUBERT** ; **M. BONNET** ; **V. BLANQUET** ; **I. CASSAR-MALEK.** The Invalidation of HspB1 Gene in Mouse Alters the Ultrastructural Phenotype of Muscles. *Plos One*, 2016, vol. 11, e0158644
- **MCBRIDE, JODI L.** ; **RYAN L. BOUDREAU** ; **SCOTT Q. HARPER** ; **PATRICK D. STABER** ; **ALEX MAS MONTEYS** ; **INÂS MARTINS** ; **BRIAN L. GILMORE** ; **HAIM BURSTEIN** ; **RICHARD W. PELUSO** ; **BARRY POLISKY.** Artificial miRNAs mitigate shRNA-mediated toxicity in the brain: Implications for the therapeutic development of RNAi. *Proceedings of the National Academy of Sciences*, 2008, vol. 105, 5868-73
- **MERLIN, SIMONE** ; **ANTONIA FOLLENZI.** Transcriptional Targeting and MicroRNA Regulation of Lentiviral Vectors. *Molecular Therapy - Methods & Clinical Development*, 2019, vol. 12, 223-32
- **OSÓRIO, LUÍSA** ; **RIK GIJSBERS** ; **MARUSELA OLIVERAS-SALVÁ** ; **ANNELIES MICHIELS** ; **ZEGER DEBYSER** ; **CHRIS VAN DEN HAUTE** ; **VEERLE BAEKELANDT.** Viral vectors expressing a single microRNA-based short-hairpin RNA result in potent gene silencing in vitro and in vivo. *Journal of Biotechnology*, 2014, vol. 169, 71-81
- **POLLARI, EVELIINA** ; **ROBERT PRIOR** ; **WIM ROBBERECHT** ; **PHILIP VAN DAMME** ; **LUDO VAN DEN BOSCH.** In Vivo Electrophysiological Measurement of Compound Muscle Action Potential from the Forelimbs in Mouse Models of Motor Neuron Degeneration. *JoVE*, 2018, e57741

- **RAUCH, J. N.** ; **E. TSE** ; **R. FREILICH** ; **S. A. MOK** ; **L. N. MAKLEY** ; **D. R. SOUTHWORTH** ; **J. E. GESTWICKI.** BAG3 Is a Modular, Scaffolding Protein that physically Links Heat Shock Protein 70 (Hsp70) to the Small Heat Shock Proteins. *J Mol Biol*, 2017, vol. 429, 128-41
- **SCHINDELIN, J.** ; **I. ARGANDA-CARRERAS** ; **E. FRISE** ; **V. KAYNIG** ; **M. LONGAIR** ; **T. PIETZSCH** ; **S. PREIBISCH** ; **C. RUEDEN** ; **S. SAALFELD** ; **B. SCHMID**. Fiji: an open-source platform for biological-image analysis. *Nat Methods*, 2012, vol. 9, 676-82
- **SCHNEIDER, C.A** ; **W.S. RASBAND** ; **K.W. ELICEIRI.** NIH Image to ImageJ: 25 years of image analysis. *Nat Methods*, 2012, vol. 9, 671-75
- **SHAN, GE** ; **YUJING LI** ; **JUNLIANG ZHANG** ; **WENDI LI** ; **KEITH E. SZULWACH** ; **RANHUI DUAN** ; **MOHAMMAD A. FAGHIHI** ; **AHMAD M. KHALIL** ; **LIANGHUA LU** ; **ZAIN PAROO**. A small molecule enhances RNA interference and promotes microRNA processing. *Nature Biotechnology*, 2008, vol. 26, 933-40
- **SHEMETOV, A. A** ; **N. B. GUSEV.** Biochemical characterization of small heat shock protein HspB8 (Hsp22)-Bag3 interaction. *Arch Biochem Biophys*, 2011, vol. 513, 1-9
- **SILVA, JOSE M.** ; **MAMIE Z. LI** ; **KEN CHANG** ; **WEI GE** ; **MICHAEL C. GOLDING** ; **RICHARD J. RICKIES** ; **DESPINA SIOLAS** ; **GUANG HU** ; **PATRICK J. PADDISON** ; **MICHAEL R. SCHLABACH**. Second-generation shRNA libraries covering the mouse and human genomes. *Nature Genetics*, 2005, vol. 37, 1281-88
- **STAVROU, M** ; **I. SARGIANNIDOU** ; **T. CHRISTOFI** ; **K. A. KLEOPA.** Genetic mechanisms of peripheral nerve disease. *Neurosci Lett*, 2021, vol. 742, 135357
- **STEGMEIER, FRANK** ; **GUANG HU** ; **RICHARD J. RICKLES** ; **GREGORY J. HANNON** ; **STEPHEN J. ELLEDGE.** A lentiviral microRNA-based system for single-copy polymerase II-regulated RNA interference in mammalian cells. *Proceedings of the National Academy of Sciences*, 2005, vol. 102, 13212-17
- **STURNER, E.** ; **C. BEHL.** The Role of the Multifunctional BAG3 Protein in Cellular Protein Quality Control and in Disease. *Front Mol Neurosci,*, 2017, vol. 10, 177
- **SUN, DAQIAN** ; **MARGHERITA MELEGARI** ; **SUNANDINI SRIDHAR** ; **CHARLES E. ROGLER** ; **LIANG ZHU.** Multi-miRNA hairpin method that improves gene knockdown efficiency and provides linked multi-gene knockdown. *BioTechniques*, 2006, vol. 41, 59-63
- **TEDESCO, B** ; **L. VENDREDY** ; **V. TIMMERMAN** ; **A. POLETTI.** The chaperone-assisted selective autophagy complex dynamics and dysfunctions. *Autophagy*, 2023, vol. 19, 1619-41
- **TOURNIER, J. D** ; **R. SMITH** ; **D. RAFFELT** ; **R. TABBARA** ; **T. DHOLLANDER** ; **M. PIETSCH** ; **D. CHRISTIAENS** ; **B. JEURISSEN** ; **C. H. YEH** ; **A. CONNELLY.** MRtrix3: A fast, flexible and open software framework for medical image processing and visualisation. *Neuroimage*, 2019, vol. 202, 116137
- **VAN DER PERREN, A.** ; **J. TOELEN** ; **M. CARLON** ; **C. VAN DEN HAUTE** ; **F. COUN** ; **B. HEEMAN** ; **V. REUMERS** ; **L. H. VANDENBERGHE** ; **J. M. WILSON** ; **Z. DEBYSER**. Efficient and stable transduction of dopaminergic neurons in rat substantia nigra by rAAV 2/1, 2/2, 2/5, 2/6.2, 2/7, 2/8 and 2/9. *Gene Therapy*, 2011, vol. 18, 517-27
- **VAN LENT, J.** ; **PRIOR, P.** ; **PÉREZ SILES, G** ; **CUTRUPI, A.N.** ; **KENNERSON, M.L** ; **VANGANSEWINKEL, T.** ; **WOLFS, E.** ; **MUKHERJEE-CLAVIN, B.** ; **JUDGE, L** ; **CONKLIN, B.** Advances and challenges in modeling inherited peripheral neuropathies using iPSCs. *Experimental Molecular Medicine*, 2024
- **XU, JIANMING.** Preparation, Culture, and Immortalization of Mouse Embryonic Fibroblasts. *Current Protocols in Molecular Biology*, 2005, vol. 70, 28.1.1-28.1.8
- **YANG, X. D** ; **Z. D. CEN** ; **H. P. CHENG** ; **K. SHI** ; **J. BAI** ; **F. XIE** ; **H. W. WU** ; **B. B. LI** ; **W. LUO.** L-3-n-Butylphthalide Protects HSPB8 K141N Mutation-Induced Oxidative Stress by Modulating the Mitochondrial Apoptotic and Nrf2 Pathways. *Front Neurosci*, 2017, vol. 11, 402